# EUROPEAN PATENT APPLICATION

(11) **EP 4 088 722 A1**
(43) Date of publication of application: **16.11.2022**
(21) Application number: 21173645.9
(22) Date of filing: 12.05.2021
(51) Int. Cl.: A61K 31/52

(54) **PHARMACEUTICAL COMPOSITIONS, USES THEREOF AND METHODS FOR TREATMENT OF GENETIC DISEASES CAUSED BY INTRONIC SPLICE-ACCEPTOR SITE MUTATIONS**

(71) Applicant: Justus-Liebig-Universität Gießen, 35390 Gießen (DE)
(72) Inventor: Tikkanen, Ritva, 60596 Frankfurt (DE); Banning, Antje, 35410 Hungen (DE)
(74) Representative: Stumpf, Peter

(57) **Abstract**

The invention is directed to uses and methods for treating human subjects who have been diagnosed with an intronic acceptor splice-site mutation that results in loss or reduction in the function of the encoded protein thus causing a disease or disorder. The invention is, at least partly, based on findings that the effects of intronic acceptor site mutations that alter the conservative intronic splicing acceptor site sequence -2A or -1G (intronic acceptor splice site "AG") resulting in loss of function of the resulting incorrectly spliced gene product, can be significantly ameliorated using small molecules that include certain methylxanthine derivatives and certain plant-derived substances thus resulting in production of the correctly spliced and functional protein in patient cells.

## Description

### BACKGROUND

Among the most difficult-to-treat gene variants in terms of small molecule therapy are those that result in splicing defects. About 9% - 15% of all known human disease-causing variants affect the consensus splice-sites. Most of the previous therapy approaches that target disease-causing splicing variants are based on antisense oligonucleotides (ASOs) that mask splicing sites and produce alternatively spliced protein variants that may be only partially functional. In Duchenne muscular dystrophy (DMD) that is caused by mutations in the gene encoding for the dystrophin protein, pathogenic gene variants are often found in exon 51, and an ASO that causes skipping of the mutated exon and results in a partly functional dystrophin protein has produced clinical improvements in DMD patients. Similarly, Nusinersen (Spinraza) is a therapeutic oligonucleotide used in spinal muscular atrophy (SMA) to induce alternative splicing of the SMN2 (Survival motor neuron 2) gene, a paralog of SMN1 that is deficient in SMA, which results in inclusion of an additional exon and expression of an alternative isoform of SMN2 protein that can partially compensate for the loss of function of SMN1 [Adams, L., Nat Rev Neurol 2017, 13, 66]. Very recently, splicing modifying ASOs were also described for the CLN3 (ceroid-lipofuscinosis, neuronal 3) gene that is deficient in the juvenile form of neuronal ceroid lipofuscinosis (JNCL). Most JNCL patients exhibit a genomic deletion that encompasses exons 7 and 8 of the CLN3 gene. The splice-modulating ASOs target exon 5 that is also excluded, resulting in a partially functional, truncated CLN3 protein due to correction of the frame-shift produced by exon 7 and 8 exclusion. In another NCL disease, an antisense splice-modulator known as Milasen for the CLN7 gene was developed for a single patient exhibiting an intronic retrotransposon. A novel exon is thus included in the mRNA, producing a frame-shifted protein variant.

Milasen masks the aberrant splice-site in the retrotransposon and thus corrects the splicing. These examples show that ASOs have significant potential for the treatment of genetic diseases involving splice variants with additional splice-sites that can be masked by the ASOs. However, they are extremely ex-pensive to develop and to use, and they require a life-long, invasive administration of the therapeutic ASO. Furthermore, ASOs are of limited use for splice variants that result in a loss of a splice-site.

Thus, splicing frequently result in a complete loss of function of the protein in question. Therapy approaches based on antisense oligonucleotides for specific gene mutations have been developed in the past, but they are very expensive and require invasive, life-long administration. Modulation of splicing by means of small molecules that could be delivered orally and would be less expensive to produce is of great interest for the therapy of genetic diseases resulting from splice-site mutations. However, only very few of such molecules have been shown to work.

Regulation of mRNA splicing has attracted attention in recent years due to its relevance for numerous human diseases, including cancer and many genetic disorders. Alternative splicing of genes is a very common process, and a vast majority of human pre-mRNAs have been suggested to be subjected to alternative splicing. Changes in splicing due to genetic mutations in the splice-sites are the underlying cause in about 15% of genetic. In some diseases such as late infantile neuronal ceroid lipofuscinosis (cLINCL), splice-site mutations may even be among the most common disease-causing gene variants. Therefore, therapy approaches that aim at correcting the splicing defect may in some cases be beneficial for a large fraction of patients. For example, the cLINCL variant analyzed in this study is present in 27% of patient alleles, making splicing therapy an attractive option in this disease.

Among many other effects, e.g., cell cycle regulation, neuroactive effects, adenosine receptor modulation, phosphodiesterase inhibition, caffeine has been shown to interfere with gene expression by either changing the expression of splicing enhancers and/or by stabilizing mRNAs containing nonsense-codons [Bode, A.M.; Dong, Z., Cancer Lett 2007, 247, 26-39]. Caffeine induces the expression of the splicing factor serine and arginine rich splicing factor 2 (SRSF2) and down-regulates the expression of SRSF3, and it was shown to regulate alternative splicing of the tumor suppressors p53 and Kruppel like factor 6 (KLF6) [Lu, G.Y.; et al., Int J Biochem Cell Biol 2014, 47, 83-92]. Splicing factors of the serine/arginine-rich protein family, such as SRSF2, bind to exonic splicing enhancers (ESE) and are able to modulate splicing by regulation of the spliceosome assembly [Howard, J.M.; Sanford, J.R., Wiley Interdiscip Rev RNA2015, 6, 93-110]. Caffeine can attenuate nonsense-mediated decay (NMD) by inhibiting the kinase SMG1, which is responsible for the phosphorylation and activation of UPF1, a main component of the classical NMD [Yamashita, A.; et al., Genes Dev 2001, 15, 2215-2228]. In cell culture studies, caffeine has been shown to improve the read-through capacity of Ataluren and aminoglycosides [Harada, N., et al., Heliyon 2019, 5]. In some epidemiological studies, caffeine exhibited moderate neuroprotective effects in neurodegenerative diseases such as Alzheimer's and Parkinson's disease [Kolahdouzan, M.; Hamadeh, M.J. CNS Neurosci Ther 2017, 23, 272-290].

Due to its bronchodilating properties, theophylline has been used for many years to treat airway diseases such as COPD (chronic obstructive pulmonary disease) and asthma, but it has been increasingly replaced by other medications that provide a better therapeutic window and less side effects [Matera, M.G.; et al., Int J Chron Obstruct Pulmon Dis 2017]. Similar to caffeine, also theophylline was shown to interfere with the expression of SRSF2, SRSF3 and alternative splicing of p53 and KLF6 [Chang, et al. Oncotarget, 2017, Vol. 8, (No. 60), pp: 101461-101474]. Theophylline may also exert its effects on gene expression by activation of histone deacetylases (HDACs) [Ito, K., et al., Proc Natl Acad Sci U S A 2002, 99, 8921-8926].

Doxofylline is a synthetic methylxanthine that contains a dioxalane group. It also shows bronchodilating properties, but has a better tolerability and an improved therapeutic window as compared to theophylline. Unlike other xanthines, doxofylline lacks affinity for adenosine receptors and does not produce stimulant effects, which might improve its suitability as a therapeutic agent [Matera, M.G., et al., Int J Chron Obstruct Pulmon Dis 2017, 12, 3487-3493]. However, there are no reports so far on an involvement of doxofylline in splicing regulation.

Pentoxifylline is a xanthine derivative that exhibits, e.g., anti-fibrotic and anti-inflammatory properties, and it is used to improve blood flow in human diseases due to its vasodilatative effects [Brie, D., et al. J Hypertens 2016, 34, 2318-2329]. Similar to caffeine and theophylline, pentoxifylline was also able to increase the expression of the serine/arginine-rich protein SRSF2 [Shi, J., et al., J Biol Chem 2015, 290, 14986-15003]. It was shown that caffeine exerts its effect on SRSF2 expression by downregulating micro-RNAs that normally repress SRSF2 expression. Enhanced SRSF2 translation then affects the splicing and stability of SRSF2 mRNA, further increasing its own expression [Id.]. However, it has not been directly shown if pentoxifylline enhances SRSF2 expression through the same mechanism.

Luteolin, an example of a flavonoid, and kinetin, an example of cytokinins, are plant-derived substances that have been shown to affect gene splicing. Interestingly, these compounds appear to have some potential in enhancing splicing at mutated splice-sites. Kinetin has been suggested to be effective in enhancing the splicing of mutated splice-donor sites [Hims, M.M., et al., J Mol Med (Berl) 2007, 85, 149-161; Slaugenhaupt, S.A., et al., Hum Mol Genet 2004, 13, 429-436; Salani, M., et al., SLAS Discov 2019, 24, 57-67; Axelrod, F.B., et al., Pediatr Res 2011, 70, 480-483], whereas luteolin has been speculated to improve splicing of weak splice-acceptor sites [Kurata, M., et al., iScience 2019, 22, 336-352; Arango, D., et al., Proc Natl Acad Sci U S A 2013, 110, E2153-2162; Chiba, M., et al.,. Chem Biol Drug Des 2016, 87, 275-282]. However, the effects have only been shown in in vitro minigene assays based on which it cannot be predicted that any of the effects could be re-produced in patient cells. Moreover, even the minigene assays have previously shown that the effect may be mutation- and or gene-specific [Chiba, M., et al., Chem Biol Drug Des 2016, 87, 275-282].

We are not aware of publications that have shown an in vivo effect of methylxanthine derivatives of plant-derived substances on increasing correct splicing and resulting in increase of protein activity that would have ameliorated a genetic disease phenotype.

Modulation and correction of specific diagnosed splicing defects that cause genetic diseases by small molecules could provide a therapy option that could be used alone or as a supplementary therapy, such as after gene therapy or along with enzyme replacement therapy, wherein molecules capable of crossing the blood-brain barrier would be particularly useful as they could be administered systemically. It would be useful to be able to predict what kinds of splice-site mutations can be addressed or targeted with specific small molecule therapies.

Accordingly, it would be useful to identify classes of compounds that could improve in vivo production of functional proteins from mutated alleles that contain specific mutations in the intronic splicing sites, and wherein the production of the encoded protein has been partially or completely lost as a result of the defective splicing that result from intronic splice-acceptor site mutations.

### SUMMARY

Using a minigene approach and patient-derived cells, the inventors have shown that by administering selected orally available small molecules, it is surprisingly possible to increase the in vivo production of the functional protein in cells where an intronic, specifically intronic acceptor site splice-site mutation has resulted in loss-of-function of the encoded protein.

The inventors have shown that the effects of intronic acceptor site mutations that alter the conservative intronic splicing acceptor site sequence -2A or -1G (intronic acceptor splice site "AG") resulting in loss of function of the resulting incorrectly spliced gene product, can be significantly ameliorated using small molecules that include certain methylxanthine derivatives and certain plant-derived flavonoids thus resulting in production of the correctly spliced and functional protein in patient cells.

The inventors demonstrated the corrective splicing effect using two different splice site mutations, namely, AG->GG and AG->AC, as examples. They showed that several small molecules were able to significantly increase the production of correctly spliced, and functional protein.

In one example, the inventors showed that a variety of methylxanthine derivatives and/or the plant-derived flavonoid luteolin enhance the correct splicing of the *AGA* mRNA transcribed from the *AGA* gene comprising a splice-site mutation c.128-2A>G in aspartylglucosaminuria and also result in increased AGA enzyme activity in patient cells demonstrating that the splicing defect correction is specific for the splice site and results in correctly spliced, functional protein in vivo. Using the same system, the inventors also showed that the same small molecules can also increase the activity of one of the most common disease-causing TPP1 gene variants in classic late infantile neuronal ceroid lipofuscinosis where the mutation in the intronic splice-acceptor site is also responsible for the original loss of function of the enzyme in the patients.

Therefore, without wishing to be bound by a theory, the data demonstrate that splice-modulation with orally available small molecules is a valid therapy option for treatment of diseases or disorders that are caused by intronic splice-acceptor site mutations, for example, splice-acceptor site mutations in -1 or -2 position of the intron, and rather than resulting in random, cryptically spliced proteins, restore the correct splicing thus resulting in increased production of the functional protein. Pharmaceutical compositions comprising these small molecules can thus be used for the treatment of patients who have been identified as being affected by a disease caused by such splice-acceptor site mutation(s). Examples of such diseases include enzyme-defects, such as lysosomal storage disorders but can also include other diseases caused by intronic acceptor splice site mutations in, e.g., membrane proteins, transmembrane proteins, and structural proteins. For example, diseases where increased activity of either an enzyme, membrane protein, transmembrane protein, or a structural protein could lead to improvement in disease pathology caused by loss of a functional protein, and wherein such loss of function results from a splice-acceptor site mutation, such as splice-acceptor site mutations in -1 or -2 position of the intron, in the patient's cells. The mutation can be, e.g., alteration of the consensus sequence "AG" to GG, AC, AT, AA, CG, or TG. In some aspects the mutation is one of "AG" to GG, AC, AT, AA, CG. In some aspects, the mutation is one of "AG" to GG, AC, AT. In some aspects, the mutation is one of "AG" to GG or AC. In some aspects, the mutation is AG->GG. In some aspects, the mutation is AG->AC. In some aspects the mutation is not AG->TG. In some aspects, the mutation results in loss of function of an enzyme, transmembrane protein or a receptor. In some aspects, the mutation results in loss of function of an enzyme. In some aspects, the mutation is not in an extracellular matrix protein or a structural protein, such as fibronectin-1.

Accordingly, provided are uses of methylxanthine derivatives and/or plant-derived flavonoids for the treatment of diseases that are caused by intronic splice-acceptor site mutations. The administration of the methylxanthine derivatives and/or plant-derived flavonoids results in increased production of a functional protein encoded by the gene disrupted by a splice-site mutation in an intronic region. Also provided are methods of treating diseases or disorders caused by intronic splice-acceptor site mutations by administering therapeutically effective amounts of methylxanthine derivatives and/or plant-derived flavonoids and/or cytokinins in a pharmaceutically acceptable carrier. Further provided are compositions comprising a combination of one or more methylxanthine derivatives and one or more plant-derived flavonoids, such as a combination of caffeine and luteolin, and their uses in treatment of diseases that are caused by intronic splice-acceptor site mutations. Methods for improving mRNA stability and/or pre-mRNA splicing using one or more methylxanthine derivatives and one or more plant-derived flavonoids are also described.

In some aspects, the patient population that can be treated includes patients who are not receiving other treatments. In some aspects, the patient population includes patients who have been treated with, e.g., gene therapy approaches or who are being treated with other methods, such as using enzyme replacement therapy or nucleotide-based medicines, such as antisense oligonucleotides or RNA-based therapies.

Accordingly, the invention provides a pharmaceutical composition comprising, as an active agent, a methylxanthine derivative, such as caffeine, pentoxifylline or theophylline, and/or a plant-derived flavonoid, for example luteolin, apigenin, or quercetin, or a combination thereof for use in the treatment of a genetic disease caused by a splice-acceptor site mutation in an intron. In some aspects, the combination comprises caffeine and luteolin. In some aspects, the combination comprises or consists of caffeine, luteolin and quercetin and/or apigenin. In some aspects, the combination is caffeine and quercetin. In some aspects the combination comprises caffeine and apigenin.

In some aspects, the splice-acceptor site mutation is in the position -1 or -2 of the acceptor site of the intron. In some aspects the mutation is one of alteration from "AG" to GG, AC, AT, AA, CG. In some aspects, the mutation is one of "AG" to GG, AC, AT. In some aspects, the mutation is one of "AG" to GG or AC. In some aspects, the mutation is AG->GG. In some aspects, the mutation is AG->AC. In some aspects the mutation is not AG->TG.

In some aspects, the mutation results in loss of function of an enzyme, transmembrane protein or a receptor. In some aspects, the mutation results in loss of function of an enzyme. In some aspects, the mutation is not in an extracellular matrix protein or a structural protein, such as fibronectin-1.

In some aspects of any use, the encoded protein is an enzyme, such as a lysosomal enzyme, such as aspartylglucosaminidase or tripeptidyl peptidase 1.

In some aspects of any use, the encoded protein is a structural protein.

In some aspects of any use, the encoded protein is a membrane protein.

In some aspects of any use or composition of the invention, the methylxanthine derivative is caffeine.

In some aspects of any use or composition of the invention, the plant-derived flavonoid is luteolin.

Also described is a method for treating a subject/patient carrying a splice-acceptor site mutation, the method comprising administering to a subject identified as carrying a splice-acceptor site mutation, a therapeutically effective amount of a methylxanthine derivative or a plant-derived flavonoid, for example, luteolin, apigenin or quercetin or a combination thereof, and a pharmaceutically acceptable carrier.

In some aspects of any method described herein, the splice-acceptor site mutation is predicted to result in loss-of-function or reduced function of the encoded protein.

In some aspects of any method described herein, the splice-acceptor site mutation is in the position -1 or -2 of the conserved acceptor site wherein position -1 is altered from G to A, C, or T, or position -2 is altered from A to C, G, or T.

In some aspects of any method described herein, the splice-acceptor site mutation is in the position -1 or -2 of the acceptor site of the intron. In some aspects the mutation is one of alteration from "AG" to GG, AC, AT, AA, CG. In some aspects, the mutation is one of "AG" to GG, AC, AT. In some aspects, the mutation is one of "AG" to GG or AC. In some aspects, the mutation is AG->GG. In some aspects, the mutation is AG->AC. In some aspects the mutation is not AG->TG.

In some aspects of any method described herein, the encoded protein is an enzyme.

In some aspects of any method described herein, the encoded protein is a structural protein.

In some aspects of any method described herein, the encoded protein is a membrane protein.

In some aspects of any method described herein, the methylxanthine derivative is caffeine, and the method comprises administering caffeine.

In some aspects of any method described herein, the therapeutically effective amount can be titrated to an amount that increases the amount of the protein in vivo.

In some aspects of any method described herein, the plant-derived flavonoid is luteolin and the method comprises administering luteolin.

In some aspects of any method described herein, the method comprises administering a combination of caffeine and luteolin.

In some aspects of any method described herein, the encoded protein is aspartylglucosaminidase (AGA).

In some aspects of any method described herein, the encoded protein is Tripeptidyl Peptidase 1 (TPP1).

In some aspects of any methods or uses described herein the patient/subject has not been diagnosed as having an autism spectrum disorder or the diagnosis of autism spectrum disorder has specifically been replaced by a diagnosis of a specific genetic disease, because the patient has been diagnosed as carrying a specific single-gene disease by nucleic acid sequence analysis, wherein a single disease-causing mutation at an intronic splice-acceptor site has been identified.

In some aspects of any method described herein, the patient/subject is treated or has been treated with enzyme-replacement therapy or gene therapy.

In one embodiment, the invention describes a pharmaceutical composition comprising a combination of plant-based flavonoid and a methylxanthine derivative and a pharmaceutically acceptable carrier. In one embodiment, the pharmaceutical composition comprises a combination of luteolin and caffeine and a pharmaceutically acceptable carrier.

### BRIEF DESCRIPTION OF DRAWINGS

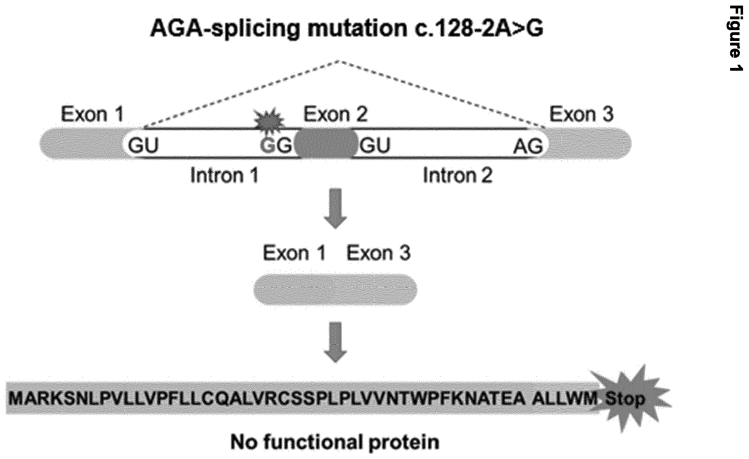

**Figure 1** shows predicted consequences of the c.128-2A>G AGU mutation.

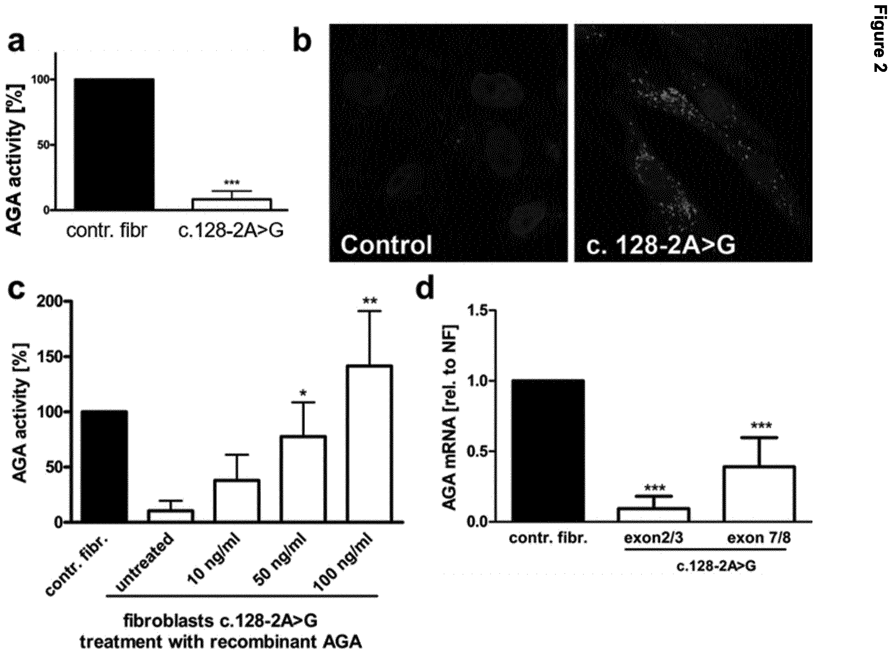

**Figures 2A-2D** demonstrate characterization of the c.128-2A>G AGU variant. In **FIG. 2A**, control and c.128-2>G AGU fibroblasts were lysed, and the AGA activity was measured fluorimetrically. Eleven independent experiments were performed. The graph shows the mean of the data ± SD. Statistical analysis was performed with unpaired *t*-test. In **FIG. 2B**, control fibroblast and the fibroblasts of the patient that were stained with Lysotracker-red to visualize lysosomal abnormalities. In **FIG. 2C**, the patient fibroblasts were treated with the indicated amounts of recombinantly expressed, Strep-tagged AGA fusion protein for 48 h, and the AGA enzyme activity was measured and compared with the control fibroblasts whose activity was set to 100%. **FIG. 2D** shows quantitative real-time PCR of AGA mRNA in patient fibroblasts with the c.128-2A>G variant. AGA transcript was detected with two different primer pairs, one for exons 2 and 3 and the other one for exons 7 and 8. The c.128-2A>G variant leads to skipping of exon 2, as indicated by the lower transcript amount with the exons 2+3 primers as compared to the primer pair for exons 7+8. Bars represent the mean ± SD of 10 independent experiments. Statistical analysis was done by 1 way ANOVA against the control fibroblasts. Values of p < 0.05 were considered significant (*) while values of p < 0.01 were considered very significant (**) and p < 0.001 extremely significant (***).

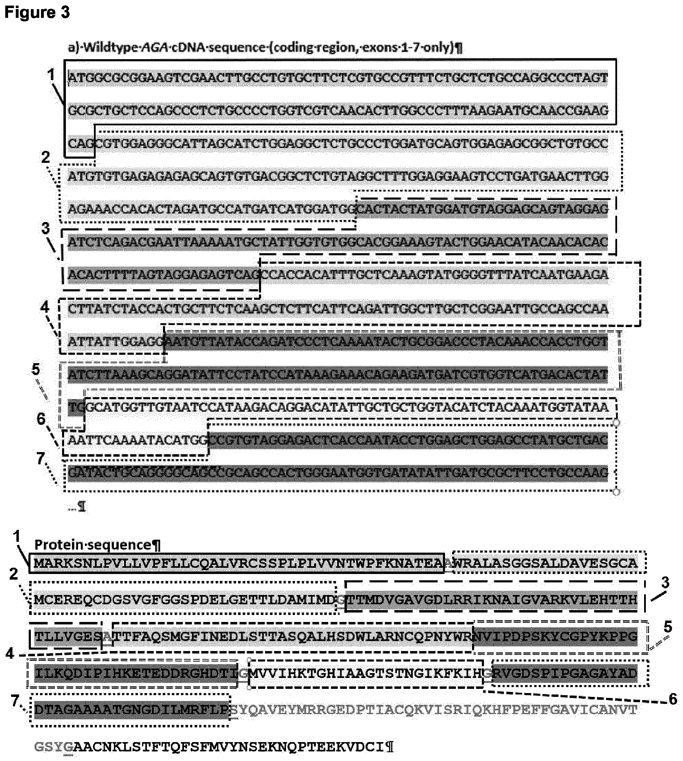

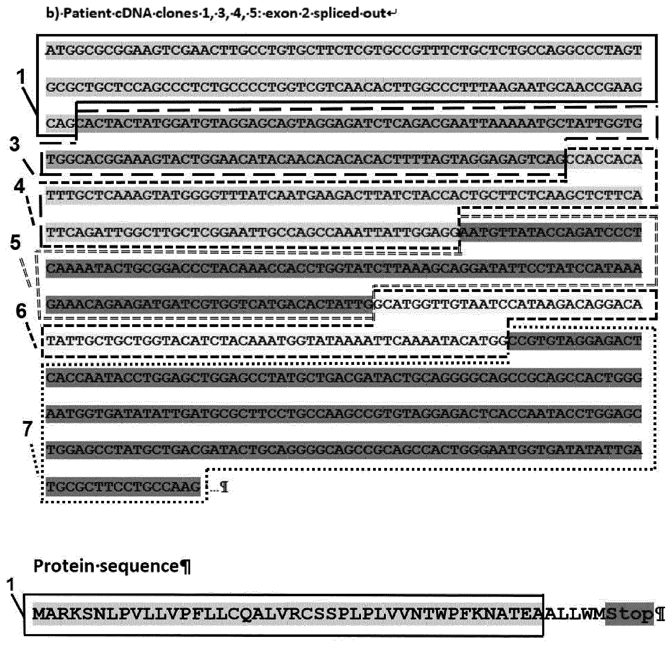

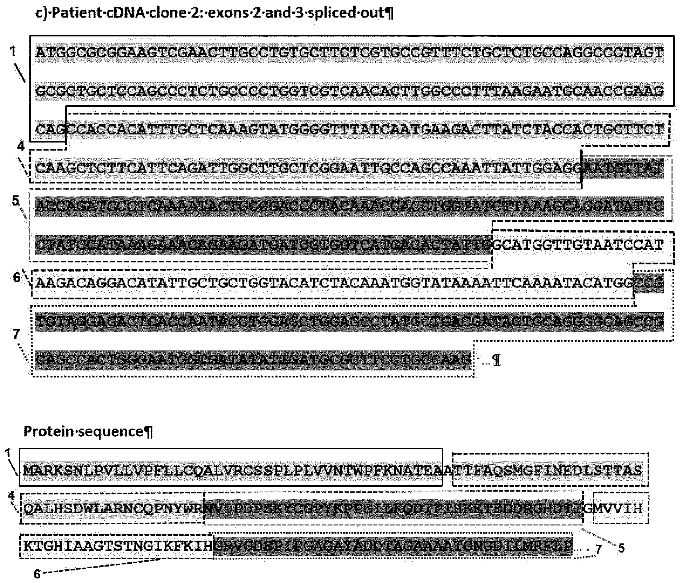

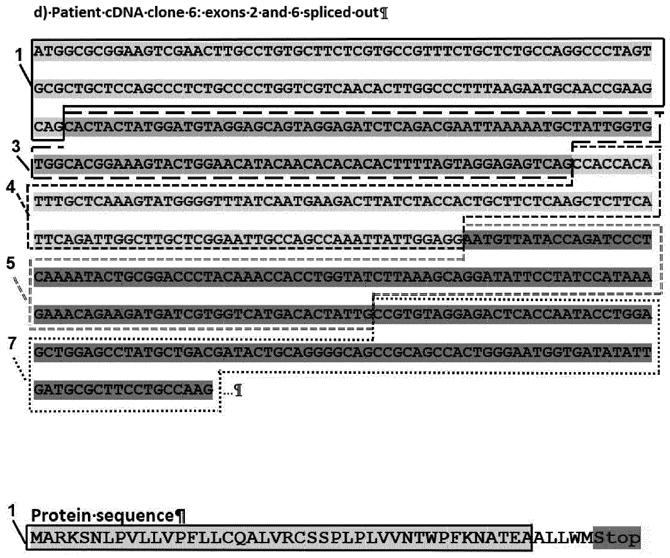

**Figures 3A-3D** show sequence analysis of the cloned AGA cDNAs from patient fibroblasts with the c.128-2A>G variant. The exons are marked in blocks with different hatching and numbering. **FIG. 3A** shows AGA reference coding sequence (NM_000027.4) and translation thereof. Individual exons are shown in greyscale. The codons for the amino acids, shown as underlined (wildtype protein sequence) at the borders of two exons contain bases from both exons. **FIGS. 3B-3D** show experiments where total RNA was isolated from patient fibroblasts, reversely transcribed into cDNA, and the AGA coding region was amplified by standard PCR. The products were cloned into pcDNA3 and six clones were sequenced. In all cases, exon 2 was missing, either alone **(****FIG. 3B****)** or in combination with exon 3 **(****FIG. 3C****)** or exon 6 **(****FIG. 3D****).** Miss-splicing leads either to introduction of an early stop codon **(****FIGS. 3B****,** **3D****)** or an in-frame deletion of 89 amino acids **(****FIG. 3C****).**
Figure 3A shows Wildtype AGA cDNA sequence (coding region, exons 1-7 only) in SEQ ID No 21. Protein Sequence to Wildtype AGA cDNA sequence (coding region, exons 1-7 only) in SEQ ID No 22.
Figure 3B shows Patient cDNA clones 1, 3, 4, 5: exon 2 spliced out in SEQ ID No 23. Protein Sequence to Patient cDNA clones 1, 3, 4, 5: exon 2 spliced out in SEQ ID No 24.
Figure 3C shows Patient cDNA clone 2: exons 2 and 3 spliced out in SEQ ID No 25. Protein Sequence to Patient cDNA clone 2: exons 2 and 3 spliced out in SEQ ID No 26.
Figure 3D shows Patient cDNA clone 6: exons 2 and 6 spliced out in SEQ ID No 27. Protein Sequence to Patient cDNA clone 6: exons 2 and 6 spliced out in SEQ ID No 28.

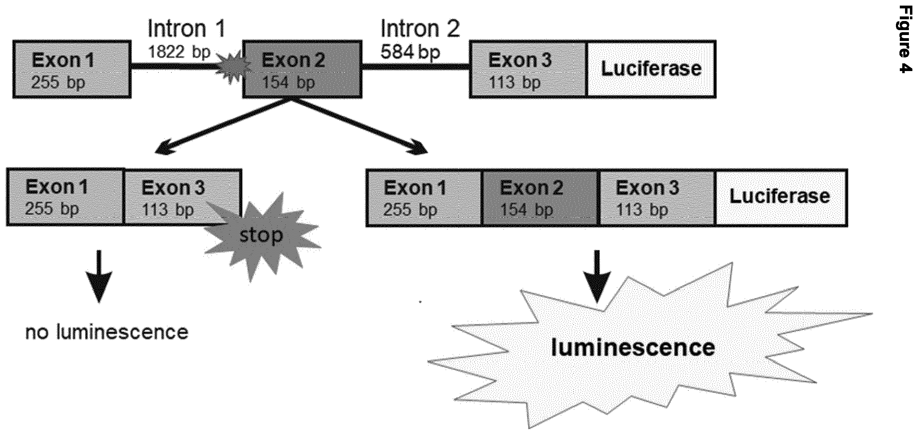

**Figure 4** shows the AGA minigene constructs with firefly luciferase. Genomic DNA was isolated from the patient fibroblasts. The region comprising exons 1-3 was amplified by standard PCR and cloned into the pcDNA3 vector. Firefly luciferase (without the ATG start codon) was cloned in-frame downstream of the AGA minigene. Skipping of exon 2 will lead to a frame shift and early termination of translation, resulting in lack of luciferase signal. The correct splicing with inclusion of all three exons produces a fusion protein containing a C-terminal luciferase protein whose activity can be measured by chemiluminescence.

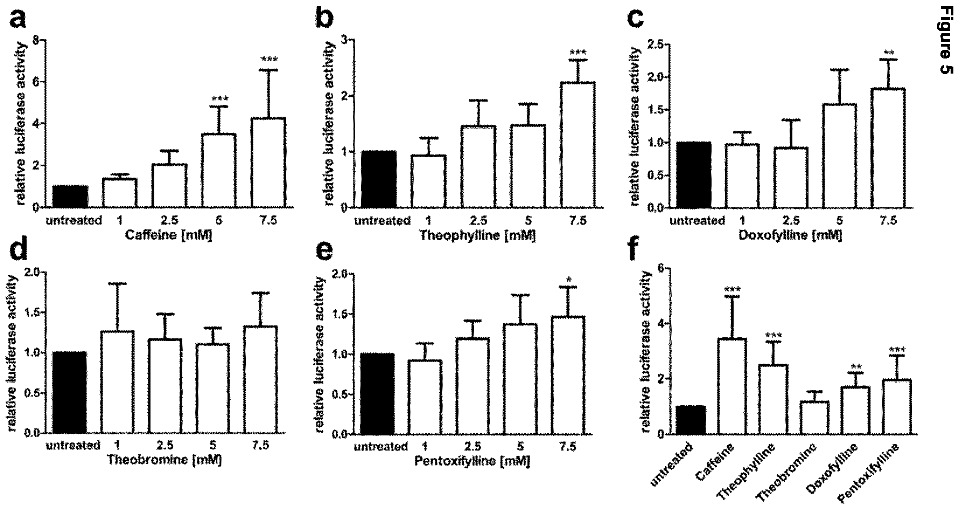

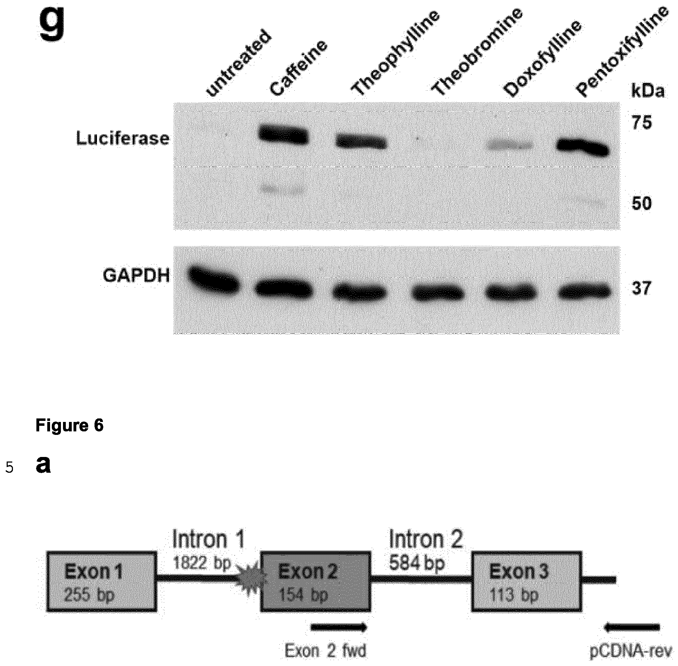

**Figures 5A-5G** show AGA-Minigene-luciferase splicing assay in HEK293T cells: Effect of methylxanthines. In **FIGS. 5A-5E****,** HEK293T cells were transfected with the AGA-minigene construct and treated with increasing amounts of the substances for 24 h. In **FIG. 5F****,** the cells were treated with 7.5 mM of each substance. Relative luciferase activity (firefly/renilla luciferase) of the untreated samples was set as 1. Bars represent the mean ± SD of at least 5 independent experiments. Statistical analysis by 1way ANOVA against the untreated samples. Values of p < 0.05 were considered significant (*) while values of p < 0.01 were considered very significant (**) and p < 0.001 extremely significant (***). In FIG. 5G, protein lysates from **FIG. 5F** were analyzed by SDS-PAGE and Western blot. Luciferase expression corresponds to the luciferase activity seen in **FIG. 5F****.**

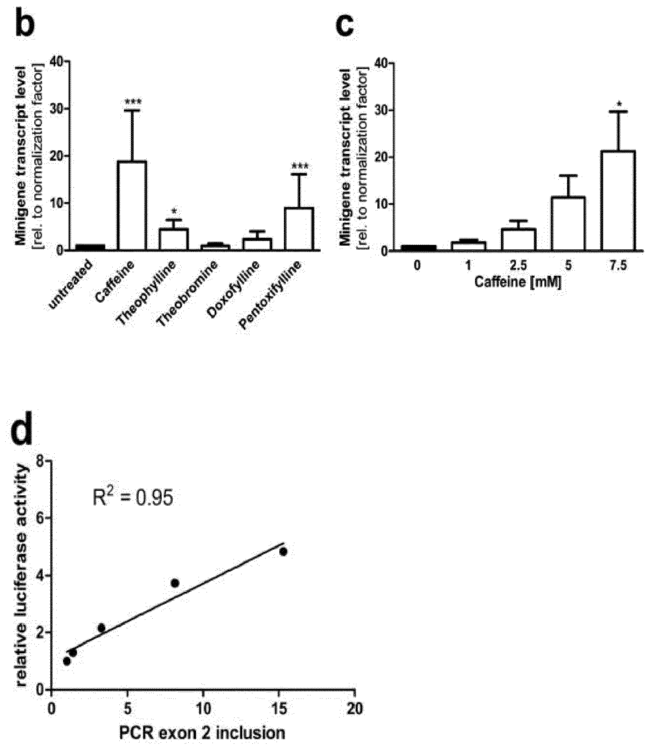

**Figures 6A-6D** show AGA minigene qRT-PCR splicing assay in HEK293T cells. In **FIG. 6A****,** the AGA minigene construct without firefly luciferase, the qRT-PCR primers for detecting exon 2 inclusion are shown with arrows. In **FIGS. 6B-6C****,** HEK293T cells were transfected with the minigene construct and treated with **(****FIG. 6B****)** 7.5 mM of the substances or **(****FIG. 6C****)** increasing doses of caffeine for 24 h. Total RNA was isolated, reversely transcribed and analyzed by qRT-PCR. Bars represent the mean ± SD of 3 independent experiments. Statistical analysis by 1 way ANOVA against the untreated samples. Values of p < 0.05 were considered significant (*) while values of p < 0.01 were considered very significant (**) and p < 0.001 extremely significant (***). In **FIG. 6D****,** the caffeine effect from luciferase assay (shown in **FIG. 5A****)** was correlated with the 7.5 mM caffeine effect in the qRT-PCR assay shown in **FIG. 6C****.**

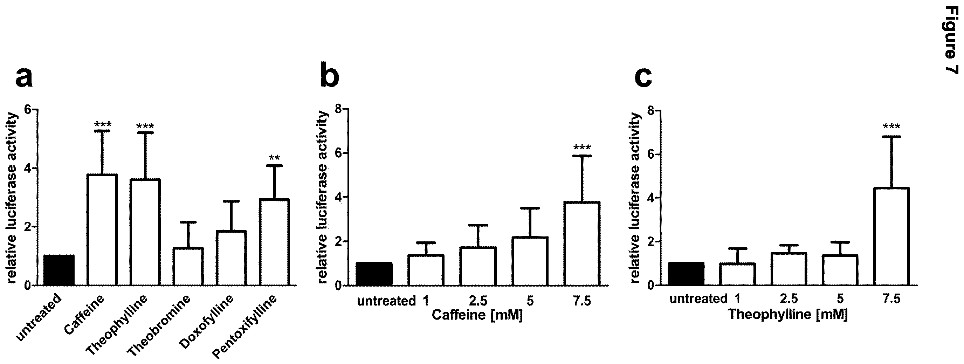

**Figures 7A-7C** show AGA luciferase minigene splicing assays in patient fibroblasts. In **FIGS. 7A-7C****,** patient fibroblasts were transfected with the AGA-luciferase minigene construct and treated with **(****FIG. 7A****)** 7.5 mM of substances or **(****FIG. 7B****)** increasing amounts of caffeine or **(****FIG. 7C****)** theophylline for 24 h. Relative luciferase activity of the untreated samples was set as 1. Bars represent the mean ± SD of at least 5 independent experiments, statistics with 1-way ANOVA against the untreated samples. Values of p < 0.05 were considered significant (*) while values of p < 0.01 were considered very significant (**) and p < 0.001 extremely significant (***).

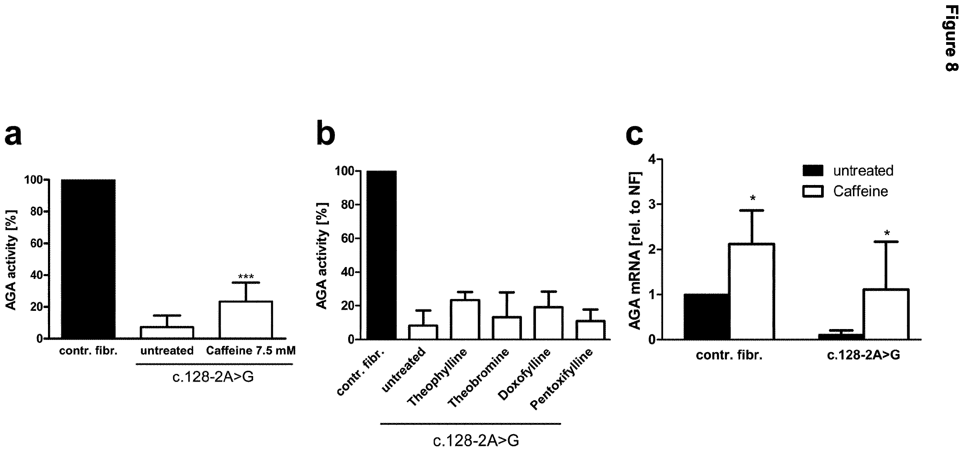

**Figures 8A-8C** show the effect of xanthine derivatives on the AGA activity and AGA mRNA level in AGU fibroblasts. Patient fibroblasts were treated for 48 h with 7.5 mM caffeine **(****FIG. 8A****)** or other methylxanthines **(****FIG. 8B****),** and the AGA activity was measured; n=4, graphs show the mean ± SD. Statistical analysis by 1way ANOVA. **(****FIG. 8C****)** Patient and control fibroblasts were treated with 7.5 mM caffeine for 24 h. AGA transcript level was measured with qPCR using primers for exons 2 and 3, n=6, 2way ANOVA. Values of p < 0.05 were considered significant (*) while values of p < 0.01 were considered very significant (**) and p < 0.001 extremely significant (***).

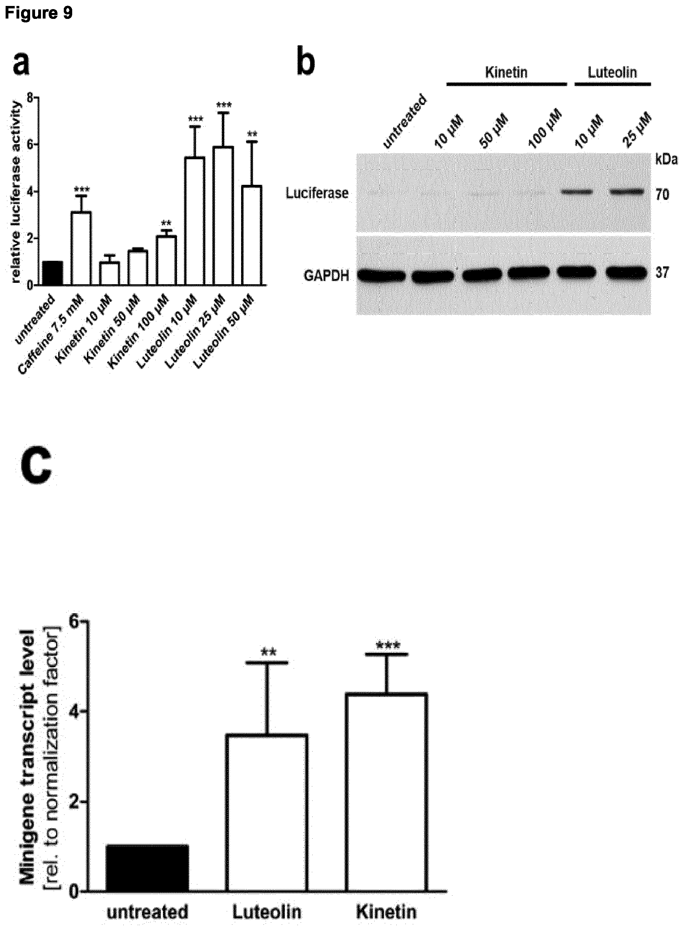

**Figures 9A-9C** show minigene splicing assays in HEK293T cells: effect of luteolin and kinetin. In **FIG. 9A****,** AGA knockout HEK293T cells were transfected with the minigene-luciferase construct and treated with increasing amounts of substances for 24 h. Relative luciferase activity of the untreated samples was set as 1. Bars represent the mean ± SD of at least 3 independent experiments, 1 way ANOVA against the untreated samples. In **FIG. 9B****,** Protein lysates from **FIG. 9A** were analyzed by SDS-PAGE and Western blot. Luciferase expression corresponds to the luciferase activity shown in **FIG. 9A****.** In **FIG. 9C****,** AGA knockout HEK293T cells were transfected with the AGA minigene construct without luciferase and treated with 25 µM luteolin or 50 µM kinetin for 24 h. Total RNA was isolated, reversely transcribed and analyzed by qRT-PCR. Bars represent the mean ± SD of 6 independent experiments, 1way ANOVA against the untreated samples. Values of p < 0.05 were considered significant (*) while values of p < 0.01 were considered very significant (**) and p < 0.001 extremely significant (***).

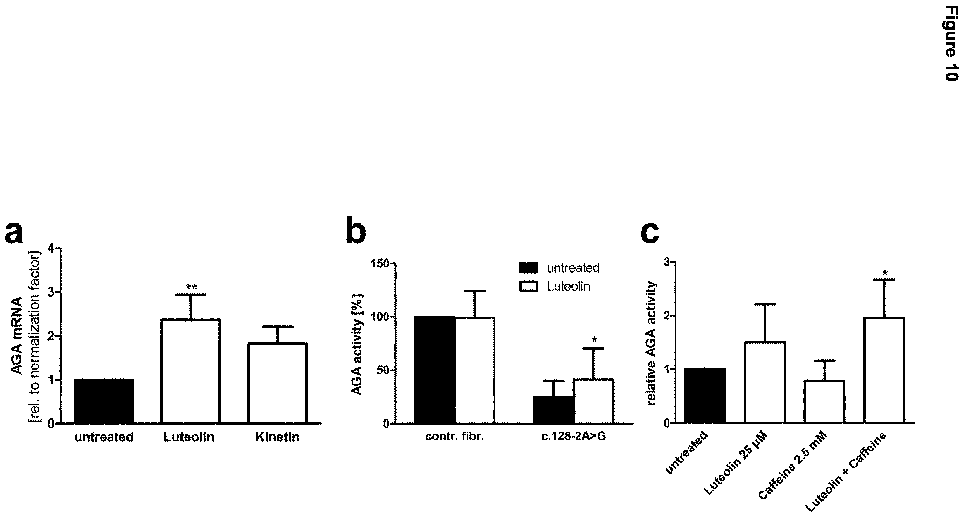

**Figures 10A-10C** demonstrate that luteolin treatment increases AGA mRNA amount and AGA activity in c.128-2A>G AGU patient fibroblasts. **FIG. 10A** shows quantitative real-time PCR of AGA mRNA with patient fibroblasts with c.128-2A>G variant after treatment with luteolin (25 µM) or kinetin (50 µM) for 24 hours. AGA was detected with the primers specific for exons 2 and 3. Bars represent the mean ± SD of 3 independent experiments, 1way ANOVA against the untreated sample. **FIG. 10B** shows treatment of fibroblasts with luteolin (25 µM) for 48 h and measurement of AGA activity with AADG as substrate, 13 independent experiments, 2way ANOVA against the untreated sample. **In** **FIG. 10C****,** patient fibroblasts were treated with suboptimal amounts of caffeine (2.5 mM) to prevent mRNA degradation, either alone or in combination with luteolin (25 µM) for 48 h. AGA activity was measured as in **FIG. 10B****,** three independent experiments, 1 way ANOVA against the untreated sample. Values of p < 0.05 were considered significant (*) while values of p < 0.01 were considered very significant (**).

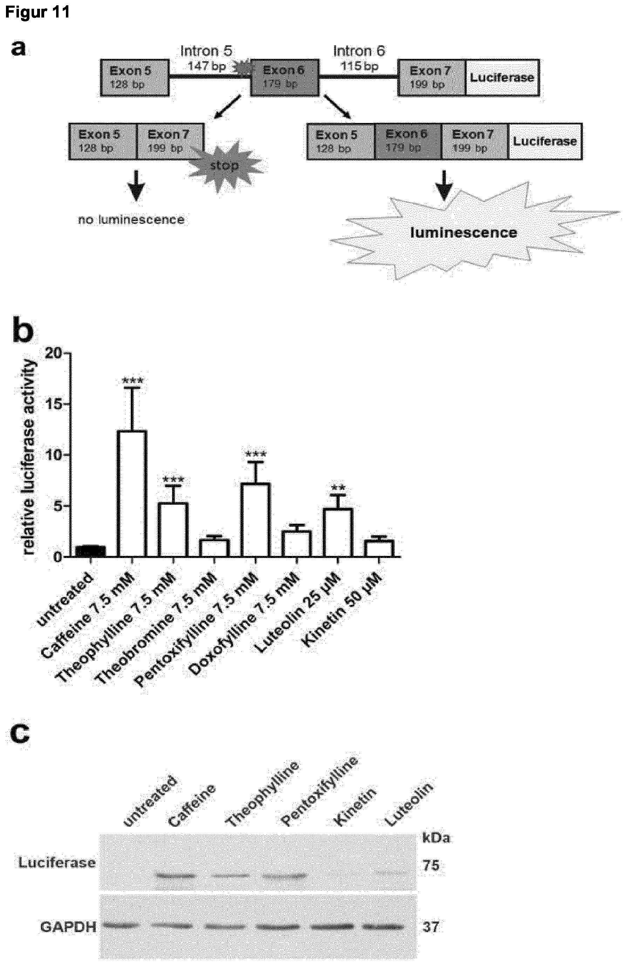

**Figures 11A-11C** show TPP1 minigene luciferase splicing assay in HEK293T cells: Effect of methylxanthines, luteolin, and kinetin. **FIG. 11A** shows a schematic drawing of the TPP1 minigene construct containing the variant c.509-1 G<C, with C-terminal firefly luciferase. In **FIG. 11B****,** HEK293T cells were transfected with the TPP1 minigene luciferase construct containing the variant c.509-1 G<C and treated with the indicated substances for 24 h. Relative luciferase activity of the untreated samples was set as 1. Bars represent the mean ± SD of at least 5 independent experiments, 1way ANOVA against the untreated samples. Values of p < 0.01 were considered very significant (**) and p < 0.001 extremely significant (***). In **FIG. 11C****,** protein lysates from **FIG. 11B** were analyzed by SDS-PAGE and western blot with an anti-luciferase antibody. Luciferase expression was observed in the samples showing significant luciferase activity in **FIG. 11B****.**
**Figures 12A-12B** show TPP1-minigene qRT-PCR splicing assays in HEK293T cells. **FIG. 12A** shows a schematic of the TPP1 minigene construct containing the variant c.509-1 G<C without firefly luciferase. The qRT-PCR primers are shown with arrows. In **FIG. 12B**, HEK293T cells were transfected with the TPP1 minigene construct and treated with caffeine (7.5 mM), luteolin (25 µM) or kinetin (50 µM) for 24 h. Total RNA was isolated, reversely transcribed and analyzed by qRT-PCR. Bars represent the mean ± SD of 3 independent experiments, 1way ANOVA against the untreated samples. Values of p < 0.01 were considered very significant (**) and p < 0.001 extremely significant (***).

### DETAILED DESCRIPTION

Described are compositions and uses of compositions that have been herein shown to increase production of functional protein from an allele that has a genetic defect on the splice-acceptor site of the intron, specifically, a mutation that alters the consensus AG-sequence at the -2 or -1 position of the acceptor splice site of an intron, and that has optionally been shown to result in loss-of-function, which causes a genetic disease. Also described are methods of treating human subjects who have been identified as carrying a mutation that alters the consensus AG-sequence at the -2 or -1 position of the acceptor splice site of an intron by administering to said human subjects an effective, protein function-increasing amount of the compounds and compositions of the invention in a pharmaceutically acceptable carrier. Combination compositions comprising one or more methylxanthine derivative with one or more plant flavonoid and a pharmaceutically acceptable carrier are also provided.

### Methylxanthine derivatives

The methylxanthine derivatives as claimed include derivatives that have an effect on specifically intronic splice-acceptor site mutations. This effect can be shown, for example, using the minigene assay as described, e.g., in this application. Examples of useful methylxanthine derivatives according to the uses, compositions and methods of the invention include caffeine, theophylline, pentoxifylline, doxofylline. In some aspects, the methods compositions and uses of the invention comprise caffeine. In some aspects, the methods compositions and uses of the invention comprise theophylline, and/or pentoxifylline. In some aspects, the methods compositions and uses of the invention comprise pentoxifylline. In some aspects, the methods compositions and uses of the invention comprise caffeine, theophylline, and/or pentoxifylline. In some aspects, the methylxanthine derivatives used in the compositions, uses or methods, specifically exclude the use of theobromine and doxofylline.

While the effect of some substances on splicing and mRNA stability, in general, has been demonstrated before, to our knowledge, no-one has previously shown or suggested that the target of these substances is specifically intronic splice-acceptor site mutations. Therefore, the data described herein leads to entirely new, unexpected and targeted uses of these substances, where the substances can be administered after identification of the particular target splice-site mutation. Specifically, we have discovered that some methylxanthine derivatives and plant-derived substances, specifically have an effect on intronic splice-acceptor site mutations and that they improve the splicing and the activity and increase the amounts of functional proteins encoded by alleles affected with intronic splice-acceptor site mutations. This finding has a significant consequence of allowing new, targeted uses for these compounds as treatment for diseases that are caused by these specific intronic splice-acceptor site mutations resulting in loss-of-function of the encoded proteins.

### Plant-derived substances

Flavonoids, a group of natural substances with variable phenolic structures, are found in fruits, vegetables, grains, bark, roots, stems, flowers, tea and wine. These natural products are well known for their beneficial effects on health and efforts are being made to isolate the ingredients, so called flavonoids. Flavonoids are now considered as an indispensable component in a variety of nutraceutical, pharmaceutical, medicinal and cosmetic applications. While these compounds have been used in general to improve health conditions, including, e.g., autism in children, to our knowledge, no-one has shown that they have a specific effect on specific splicing errors. We have discovered that some flavonoids, such as luteolin, have an effect on intronic splice-acceptor site mutations and that they improve the activity and increase the amounts of proteins encoded by alleles affected with intronic splice-acceptor site mutations. This finding has a significant consequence of allowing new, targeted uses for flavonoids, such as luteolin, apigenin, or quercetin, as treatment for diseases which are caused by intronic splice-acceptor site mutations which result in loss-of-function of the encoded proteins.

Cytokinins are phytohormones that regulate plant growth, development and senescence. Experiments both in vitro and in vivo demonstrate that they also have diverse effects on animal cells and tissues. For example, they have been shown to protect cells against various forms of stress and prevent some detrimental effects of cell aging. For example, human skin fibroblasts cultured in the presence of kinetin or trans-zeatin retain some characteristics of cells of lower passage. In another example, kinetin (6-furfurylaminopurine) has been shown to increase the lifespan of invertebrates.

### Pharmaceutical composition

The term "pharmaceutical composition" refers to a composition comprising at least one active agent and at least one pharmaceutically acceptable carrier. A pharmaceutical composition is generally formulated and administered to exert a pharmaceutically useful effect while minimizing undesirable side effects.

### Pharmaceutically acceptable carrier

The term "pharmaceutically acceptable carrier" refers to buffers, carriers, and other excipients suitable for use in contact with tissues of humans and/or animals without excessive toxicity, allergic response, irritation, or other problem or complication, commensurate with a reasonable benefit/risk ratio. The carrier(s) should be "acceptable" in the sense of being compatible with the other ingredients of the formulations and not deleterious to the recipient. Pharmaceutically acceptable carriers include buffers, solvents, dispersion media, coatings, isotonic and absorption delaying agents, and the like, that are compatible with pharmaceutical administration. The carrier may further include plant extracts. Suchcarriers, in general are well known in the art.

### Therapeutically effective amount

Generally, the therapeutically effective amount of any of the compounds of the invention can be titrated to the patient using well-known methods by medical doctors by evaluating the effect of the treatment, such as using gradual increase of the amount of the therapeutic agent. When treating, e.g., enzyme defects, the patients are typically pediatric patients and the effective amount may vary substantially compared to that used in adult population. A skilled physician will be able to titrate a therapeutically safe and effective amount using available knowledge of these compounds where they have been used for treatment of other conditions. Some considerations for therapeutic amounts and their ranges can be found, e.g., in Gal P. J Pediatr Pharmacol Ther. 2009 Apr-Jun; 14(2): 66-74 and Taliou et al. Clin Ther. 2013 May;35(5):592-602.

Some examples of therapeutically effective amounts useful in the methods and uses of the invention are provided below. A skilled doctor can further determine additional suitable amounts of the compounds and compositions by monitoring the effect of the compounds to the amount of the function of the protein that is affected by the splicing defect.

Caffeine: therapeutically effective amount can be, e.g., 1-13 mg/kg of caffeine, such as 1-10 mg/kg, for example 1-5 mg/kg of caffeine. These amounts can be used to obtain a therapeutic serum concentration of e.g., 5-50 mg/L, for example, 10-50 mg/L, for example 10-30 mg/L, or for example 5-20 mg/L.

Theophylline: therapeutically effective amount can be, e.g., 1-4 mg/kg, for example, 1-1.5 mg/kg or 2-2.5 mg/kg. These amounts can be used to obtain a therapeutic serum concentration of 4-16 mg/L, 6-10 mg/L or 10-16 mg/L.

Luteolin ((3',4',5,7-tetrahydroxyflavone): therapeutically effective amount can be e.g., 5-20 mg/kg, for example about 10 mg/kg or 7 mg/kg.

Quercetin: therapeutically effective amount can be e.g., 5-20 mg/kg, for example about 10 mg/kg or 7 mg/kg.

A composition with both caffeine and luteolin can comprise, e.g., 10-50 mg of caffeine and 50-100 mg of luteolin, or 5-10 mg of caffeine and 50-100 mg of luteolin.

### Treatment or treating

The terms "treating" or "treatment" of a disease, condition or symptom refers to obtaining therapeutic and/or prophylactic benefit, including alleviating, ablating, ameliorating, or preventing a disease, condition or symptoms, preventing additional symptoms, ameliorating or preventing the underlying metabolic causes of symptoms, inhibiting the disease or condition, e.g., arresting or slowing down the development of the disease or condition, relieving the disease or condition, causing regression of the disease or condition, relieving a condition caused by the disease or condition, or stopping the symptoms of the disease or condition.

### Subject/Patient

The terms "patient" and "subject" are used interchangeably herein and refer to human subjects. In all aspects of the invention, the subject has been diagnosed as carrying a mutation in his/her/their genomic DNA, wherein the mutation alters an intronic splice-acceptor site that results in a loss of function of the encoded protein which causes a disease or disorder in the subject. In some aspects of all the uses and methods of the invention, the compositions are administered to a patient/subject who has been diagnosed as having a splice-acceptor site mutation in location -1 or -2 in an enzyme-encoding, such as lysosomal enzyme-encoding nucleic acid sequence. In some aspects of all the methods and uses of the invention, the patient has also been diagnosed as having loss of function/significant reduction in the amount of that same enzyme using any of the well-known diagnostic laboratory tests.

In some aspects of all the methods and uses of the invention, the subject/patient has been diagnosed with a lysosomal storage disease, for example aspartylglucosaminuria or classic late infantile neuronal ceroid lipofuscinosis. These diseases can be diagnosed with routine and well-known nucleic acid analysis techniques, such as genomic sequencing of a genomic nucleic acid sample taken from the patient.

In some aspects of all methods and uses, the subject/patient is not additionally affected with bronchodilation for chronic lung disease, is not in need of prevention of intubation or assistance with extubation from mechanical ventilation, and is not affected with apnea of prematurity, and does not need treatment for renal dysfunction.

The patient can be adult, teenager or a pediatric subject/patient, including neonates. In some aspects of all methods and uses the patient is a pediatric patient.

In some aspects of any of the methods and uses, the patient has not been diagnosed with autism spectrum disorder. In some aspects or any methods and uses, an autism spectrum disorder has specifically been ruled out as a diagnosis or changed into a more specific diagnosis of a genetic disease on the basis of genetic analysis that has identified an intronic splice acceptor site mutation in the subject's genomic DNA as the cause of the patient's symptoms, even if some of the symptoms resemble autism spectrum symptoms.

Autism spectrum disease or disorder (ASD) can be diagnosed using clinical criteria. Doctors look at the child's developmental history and behavior to make a diagnosis. ASD can sometimes be detected at 18 months or younger.

The autism spectrum disease diagnostic is typically made based on the patient's behavioral symptoms. To meet diagnostic criteria for ASD according to The American Psychiatric Association's Diagnostic and Statistical Manual, Fifth Edition (DSM-5), a child must have persistent deficits in each of three areas of social communication and interaction plus at least two of four types of restricted, repetitive behaviors (see, American Psychiatric Association. Diagnostic and statistical manual of mental disorders. 5th ed. Arlington, VA: American Psychiatric Association; 2013.) Persistent deficits in social communication and social interaction across multiple contexts, as manifested by the following, currently or by history (examples are illustrative, not exhaustive; see text):
Deficits in social-emotional reciprocity, ranging, for example, from abnormal social approach and failure of normal back-and-forth conversation; to reduced sharing of interests, emotions, or affect; to failure to initiate or respond to social interactions. Deficits in nonverbal communicative behaviors used for social interaction, ranging, for example, from poorly integrated verbal and nonverbal communication; to abnormalities in eye contact and body language or deficits in understanding and use of gestures; to a total lack of facial expressions and nonverbal communication. Deficits in developing, maintaining, and understand relationships, ranging, for example, from difficulties adjusting behavior to suit various social contexts; to difficulties in sharing imaginative play or in making friends; to absence of interest in peers.

### Splice-acceptor site mutation

The mature mRNA contains only exonic sequences-the intronic ones are removed from the transcript during the splicing process. This process is held in nucleus and is dependent from the presence and interaction between the so-called cis and trans elements. The cis elements are the DNA sequences that define exons, introns, and other regulatory sequences necessary for proper splicing. They are termed consensus splice site sequences and include donor (5') and acceptor (3') splice sites, branch point and polypyrimidine tract sequences, and auxiliary cis elements including splicing silencers and enhancers.

The cis elements are the DNA sequences that include donor (5') and acceptor (3') splice sites, branch point and polypyrimidine tract sequences, and splicing silencers and enhancers. Donor and acceptor sites are evolutionary conserved and are usually defined by GT and AG nucleotides at the 5' and 3' ends of the intron, respectively. The intronic splice-acceptor site mutations as described herein refer to the site with the conserved sequence of "AG" at the 3' splice site of the intron, i.e., the site that borders the 5' end of the following exon.

A splice site mutation is a mutation in the genomic DNA that results in aberrant expression of the gene. It can be caused by a number of nucleic acid changes at typically conservative intronic regions. It can be a point-mutation, an insertion or a deletion of ta nucleotide at the conserved region involved in splicing, i.e, in the specific site of a gene at which splicing of introns out of the pre-mRNA takes place during the processing of precursor messenger RNA into mature messenger RNA.

The splice-acceptor site mutation as referred to herein refers to the site at the 3' end of an intron in the splicing of RNA. The site in the -1 or -2 position refers to the position -1 or -2 in the intron, as counted from the 5' end of the exon following the intron in question. That typically means that either the A or the G at the 3' end of the intron has been mutated and thus results in aberrant splicing of the intronic sequence. The typical mutations in this site include change of A in -2 position into G, C, or T or change of G in -1 position into C, T, or A.

In some aspects of any method described herein, the splice-acceptor site mutation is in the position -1 or -2 of the acceptor site of the intron. In some aspects the mutation is one of alteration from "AG" to GG, AC, AT, AA, CG. In some aspects, the mutation is one of "AG" to GG, AC, AT. In some aspects, the mutation is one of "AG" to GG or AC. In some aspects, the mutation is AG->GG. In some aspects, the mutation is AG->AC. In some aspects the mutation is not AG->TG.

### Loss of Function and reduced function

As referred to herein, loss of function refers to complete loss of function of a protein and reduced function or activity refers to reduction of the activity or amount of the protein by at least 50% compared to a non-mutant protein. Loss or reduction in function can result from an intronic splice-acceptor site mutation that causes, for example, an early termination of the translated protein, partial or complete loss of an exon that results in aberrant folding of the protein and subsequent loss in activity. Loss of function can also be a result from reduced amount of the encoded protein, where the mutated allele results in no production of the protein.

### Enzymes:

Enzymes are proteins that act as a catalyst in living organisms, regulating the rate at which chemical reactions proceed without itself being altered in the process.

### Lysosomal enzymes

Lysosomes are acidic intracellular membrane vesicles that contain digestive enzymes, such as glycosidases, proteases and sulfatases. Lysosomal enzymes as referred to herein are enzymes that are synthesized in the endoplasmic reticulum (ER), are transported to the Golgi apparatus, and are usually tagged for targeting to lysosomes by the addition of mannose-6-phosphate label. Reduction or lack of lysosomal enzymes is a cause of several lysosomal storage diseases.

Examples of lysosomal enzymes include:

| **Enzymes** | **Substrate** |
|---|---|
| Phosphates | |
| A- Acid phosphatase | Most phosphomonoesters |
| B-Acid phosphodiesterase | Oligonucleotides and phosphodiesterase |
| Nucleases | |
| A- Acid ribonuclease | RNA |
| B-Acid deoxyribonuclease | DNA |
| Polysaccharides/ mucopolysaccharides hydrolyzing enzymes | |
| A- beta Galactosidase | Galactosides |
| B-alfa Glucosidase | Glycogen |
| C- alfa Mannosidase | Mannosides, glycoproteins |
| D- beta Glucoronidase | Polysaccharides and mucopolysaccharides |
| E- Lysozymes | Bacterial cell walls and mucopolysaccharides |
| F- Hyaluronidase | Hyaluronic acids, chondroitin sulphates |
| H-Arylsulphatase | Organic sulfates |
| Proteases | |
| A- Cathepsin(s) | Proteins |
| B-Collagenase | Collagen |
| C-Peptidase | Peptides |
| Lipid degrading enzymes | |
| A-Esterase | Fatty acyl esters |
| B- Phospolipase | Phospholipids |

### Structural proteins

Structural proteins are the proteins that are generally fibrous and stringy. They are the most abundant class of proteins in nature. Their main function is to provide mechanical support. Examples of structural proteins include keratin, collagen, and elastin.

### Membrane proteins

Membrane proteins are common proteins that are part of, or interact with, biological membranes. Membrane proteins fall into several broad categories depending on their location. Integral membrane proteins are a permanent part of a cell membrane and can either penetrate the membrane (transmembrane) or associate with one or the other side of a membrane (integral monotopic). Peripheral membrane proteins are transiently associated with the cell membrane.

Membrane proteins perform a variety of functions vital to the survival of organisms, and include membrane receptor proteins that relay signals between the cell's internal and external environments; and transport proteins that move molecules and ions across the membranes. Membrane enzymes may have many activities, such as oxidoreductase, transferase or hydrolase. Cell adhesion molecules allow cells to identify each other and interact. For example, proteins involved in immune response. The localization of proteins in membranes can be predicted reliably using hydrophobicity analyses of protein sequences, i.e. the localization of hydrophobic amino acid sequences.

Membrane proteins are common, and medically important, as about a third of all human proteins are membrane proteins, and these are targets for more than half of all drugs.

In some aspects of the methods and uses the target disease or disorder is not caused by an acceptor splice site mutation in fibronectin-1.

### Description

The inventors have dissected the effect of several orally available small molecule compounds on splicing of pre-mRNAs that carry splice-acceptor site mutations. The tested compounds have been shown to have a beneficial effect on either mRNA stability and/or pre-mRNA splicing. We here show that some derivatives of xanthine, for example, caffeine, and the plant-derived flavonoid luteolin are able to significantly correct the splicing of AGA pre-mRNA mutated at intronic position -2 preceding exon 2 in a minigene splicing assay. In addition, caffeine and luteolin significantly increased the amount of correctly spliced AGA mRNA and AGA enzyme activity in patient fibroblasts homozygous for the said gene defect. Importantly, our data also show that the splicing of the TPP1 gene deficient in another lysosomal storage disorder, cLINCL, can be improved in a minigene assay using the same substances. Our findings demonstrate that these substances can provide a relevant therapeutic intervention for diseases that result from splice-acceptor site mutations.

The AGU patient who was part of this study is homozygous for a novel gene variant that has so far not been reported in other AGU patients. The parents originate from the same geographical region in India, but they were found not to be related to each other. Therefore, although AGU has been assumed to be extremely rare outside of Finland, this may imply that the gene variant found in our patient may be more common in this specific region in India, suggesting a founder effect, similarly to the AGU_{Fin-major} -variant in Finland. Therefore, it might be possible to identify further AGU patients in this region in India.

Our data show, surprisingly, that caffeine and luteolin, or a combination thereof, is the most promising therapy option for the AGU splice variant described in our study. In addition, theophylline and pentoxifylline also produced significant results in the AGA minigene assay. Based on these results, it can be expected that apigenin and quercetin have an effect similar to luteolin. The effect of these substances on splicing, in general, and mRNA stability, in general has been demonstrated before but thus far, no-one has shown or suggested that the target of these substances is intronic splice-acceptor site mutations.

In addition, our data suggest that theophylline can ameliorate the specific acceptor-site splicing defects we studied. However, due to its potential side effects on the heart, kidney and lung function as well as gastrointestinal effects, theophylline may not be suitable for a long-term therapy in pediatric patients, although it could be used in smaller amounts as an adjuvant to another molecule, such as luteolin or on a short-term basis, for example, while waiting for gene therapy.

Doxofylline is a synthetic methylxanthine that contains a dioxalane group. It also shows bronchodilatating properties, but has a better tolerability and an improved therapeutic window as compared to theophylline. Unlike other xanthines, doxofylline lacks affinity for adenosine receptors and does not produce stimulant effects, which might improve its suitability as a therapeutic agent. However, there are no reports so far on an involvement of doxofylline in splicing regulation. While we anticipated an effect by doxofylline, it consistently failed to exert any significant effects on splicing of the *AGA* and *TPP1* minigenes in our study.

Pentoxifylline is a xanthine derivative that exhibits e.g., anti-fibrotic and anti-inflammatory properties, and it is used to improve blood flow in human diseases due to its vasodilatative effects [Annamaraju, P.; Baradhi, K.M. Pentoxifylline. In StatPearls, Treasure Island (FL), 2020, Brie, D., et al., J Hypertens 2016, 34, 2318-2329]. Similar to caffeine and theophylline, pentoxifylline was also able to increase the expression of the serine/arginine-rich protein SRSF2 [Shi, J., et al., J Biol Chem 2015, 290, 14986-1500]. It has been shown that caffeine exerts its effect on SRSF2 expression by downregulating micro-RNAs that normally repress SRSF2 expression. Enhanced SRSF2 translation then affects the splicing and stability of SRSF2 mRNA, further increasing its own expression [Id.]. However, the prior study did not show that pentoxifylline increases SRSF2 expression.

Luteolin (an example of flavonoids) and kinetin (an example of cytokinins) are plant-derived substances that have been shown to affect gene splicing. Interestingly, these compounds appear to have distinct potential in enhancing splicing at mutated splice-sites. Kinetin has been suggested to be effective in enhancing the splicing of mutated splice-donor sites, whereas luteolin has been suggested to improve splicing of weak splice-acceptor sites. We demonstrated that kinetin did not work to ameliorate the splice-acceptor site defects as it showed no systematic positive effect on the splicing of the AGA and TPP1 variants analyzed in our study.

Our results show that the treatment effect of luteolin and caffeine and the combination of the two was surprisingly significant for the AG->GG mutation, and also for the AG->AC mutation. This was contrary to what was exemplified, e.g., in Figure 3 of Chiba et al. (Chem Biol Drug Des 2016, 87, 275-282), where luteolin and apigenin had a notable effect only on mutation from AG->TG at the intronic acceptor site, and where other mutations in the splice site responded only marginally. Moreover, while Chiba et al. speculated the effect of luteolin on splicing, they did not demonstrate that the treatment could result in correctly spliced, functional protein in patient's cell.

To our surprise, in the present study, luteolin, especially in combination with a low dose of caffeine, significantly increased correct splicing of AGA mRNA and AGA activity in AGU patient cells homozygous for the c.128-2A>G AGA gene variant. Luteolin is a plant-derived bioactive flavonoid with multiple biological effects in cell culture studies, such as anti-inflammatory, anti-allergic and anti-carcinogenic effects.

High uptake of luteolin has been shown to be associated with health benefits such as lower risk of acute myocardial infarction and ovarian cancer. Luteolin has been tested in clinical studies involving pediatric patients with autism spectrum disease. Oral application of capsules containing luteolin and quercetin, which is another flavonoid, was effective in reducing aberrant behavior and in increasing adaptive functioning in pediatric patients with autism spectrum disorder, and no major adverse effects, except for temporary irritability, were observed. However, these studies have not connected the positive effects of luteolin with altered splicing. On the other hand, luteolin and the related compound apigenin have been shown to enhance splicing at non-canonical, weak splice acceptor sites and to alter the splicing patterns of some genes. Furthermore, luteolin was identified in a screen for molecules that correct splicing at mutated splice acceptor sites.

We have demonstrated that luteolin results in surprisingly clear effect in ameliorating specifically the intronic -1 and -2 splice-acceptor site mutations. Therefore, luteolin clearly exhibit potential as a splice modulator that can be used for therapy of diseases resulting from gene variants that affect splicing. Based on these results it is reasonable to expect that quercetin would have a similar effect.

Our assay using the specific minigene described herein can be used to identify, additional flavonoids and methylxanthine derivatives that have an effect on the specific -1 and -2 splice-acceptor site mutations.

In one embodiment, the invention provides a pharmaceutical composition comprising a methylxanthine derivative, such as caffeine, pentoxifylline or theophylline, and/or a plant-derived flavonoid, such as luteolin or apigenin or quercetin, or a combination thereof for use in the treatment of a genetic disease determined to be caused by a splice-acceptor site mutation in an intron.

In some aspects of all the uses and compositions described herein, the combination is caffeine with luteolin. In some aspects of all the uses and compositions described herein, the combination is caffeine with apigenin. In some aspects of all the uses and compositions described herein, the combination is caffeine with quercetin. In some aspects of all the uses and compositions described herein, the combination is caffeine and luteolin and quercetin or apigenin.

In some aspects of all the uses and compositions described herein, the combination is theophylline with luteolin. In some aspects of all the uses and compositions described herein, the combination is theophylline with apigenin. In some aspects of all the uses and compositions described herein, the combination is theophylline with quercetin. In some aspects of all the uses and compositions described herein, the combination is theophylline and luteolin and quercetin or apigenin.

In some aspects of all the uses and compositions described herein, the combination is pentoxifylline with luteolin. In some aspects of all the uses and compositions described herein, the combination is pentoxifylline with apigenin. In some aspects of all the uses and compositions described herein, the combination is pentoxifylline with quercetin. In some aspects of all the uses and compositions described herein, the combination is pentoxifylline and luteolin and quercetin or apigenin.

In some aspects of all the uses described herein, the splice-acceptor site mutation is in the position -1 or -2 of the acceptor site of the intron.

In some aspects of all the uses described herein, the splice-acceptor site mutation is in the position -1 of the acceptor site of the intron and has altered the -1 position from nucleotide G into C, A or T.

In some aspects of all the uses described herein, the splice-acceptor site mutation is in the position -1 of the acceptor site of the intron and has altered the -1 position from nucleotide G into C or A.

In some aspects of all the uses described herein, the splice-acceptor site mutation is in the position -1 of the acceptor site of the intron and has altered the -1 position from nucleotide G into C.

In some aspects of all the uses described herein, the splice-acceptor site mutation is in the position -2 of the acceptor site of the intron and has altered the -2 position from nucleotide A into G, C or T.

In some aspects of all the uses described herein, the splice-acceptor site mutation is in the position -2 of the acceptor site of the intron and has altered the -2 position from nucleotide A into G or C.

In some aspects of all the uses described herein, the splice-acceptor site mutation is in the position -2 of the acceptor site of the intron and has altered the -2 position from nucleotide A into G.

In some aspects of all the uses described herein, the encoded protein is an enzyme, such as a lysosomal enzyme, such as aspartylglucosaminidase or tripeptidyl peptidase 1.

In some aspects of all the uses described herein, the encoded protein is a structural protein.

In some aspects of all the uses described herein, the encoded protein is a membrane protein.

In some aspects of all the uses and compositions described herein, the methylxanthine derivative is caffeine.

In some aspects of all the uses and compositions described herein, the plant-derived flavonoid is luteolin.

In some aspects of all the uses and compositions described herein, the composition comprises a combination of caffeine and luteolin.

Also described is a method for treating a subject carrying a splice-acceptor site mutation, the method comprising administering to a subject identified as carrying a splice-acceptor site mutation, a therapeutically effective amount of a methylxanthine derivative or a plant-derived flavonoid, such as luteolin or apigenin or quercetin or a combination thereof, and a pharmaceutically acceptable carrier.

In some aspects of all the methods described herein, the splice-acceptor site mutation is predicted to result in loss-of-function or reduced function of the encoded protein.

In some aspects of all the methods described herein, the splice-acceptor site mutation is in the position -1 or -2 of the acceptor site.

In some aspects of all the methods described herein, the splice-acceptor site mutation is in the position -1 of the acceptor site of the intron and has altered the -1 position from nucleotide G into C, A or T.

In some aspects of all the methods described herein, the splice-acceptor site mutation is in the position -1 of the acceptor site of the intron and has altered the -1 position from nucleotide G into C or A.

In some aspects of all the methods described herein, the splice-acceptor site mutation is in the position -1 of the acceptor site of the intron and has altered the -1 position from nucleotide G into C.

In some aspects of all the methods described herein, the splice-acceptor site mutation is in the position -2 of the acceptor site of the intron and has altered the -2 position from nucleotide A into G, C or T.

In some aspects of all the methods described herein, the splice-acceptor site mutation is in the position -2 of the acceptor site of the intron and has altered the -2 position from nucleotide A into G or C.

In some aspects of all the methods described herein, the splice-acceptor site mutation is in the position -2 of the acceptor site of the intron and has altered the -2 position from nucleotide A into G.

In some aspects of all the methods described herein, the encoded protein is an enzyme.

In some aspects of all the methods described herein, the encoded protein is a structural protein.

In some aspects of all the methods described herein, wherein the encoded protein is a membrane protein.

In some aspects of all the methods described herein, the methylxanthine derivative is caffeine, and the method comprises administering caffeine.

In some aspects of all the methods described herein, the plant-derived flavonoid is luteolin and the method comprises administering luteolin.

In some aspects of all the methods described herein, the method comprises administering a combination of caffeine and luteolin.

In some aspects of all the methods described herein, the encoded protein is aspartylglucosaminidase (AGA).

In some aspects of all the methods described herein, the encoded protein is Tripeptidyl Peptidase 1 (TPP1).

In some aspects of all the methods described herein, the subject is treated or has been treated with enzyme-replacement therapy or gene therapy.

In some aspects of all the methods described herein, if the subject has been originally diagnosed with autism spectrum disease the diagnosis has been corrected after a genetic analysis of the subject's genomic DNA to a disease resulting from a loss of function of a protein wherein the loss of function is a result of a mutation in an intronic splice-acceptor site in a gene encoding the protein.

In one embodiment, the invention provides a pharmaceutical composition comprising, as an effective agent, a combination of a plant-derived flavonoid, such as, luteolin, apigenin or quercetin, and caffeine and a pharmaceutically acceptable carrier. In one embodiment, the composition comprises, as the pharmaceutically effective agent, a combination of luteolin and caffeine and a pharmaceutically acceptable carrier. In one aspect, the combination comprises luteolin and caffeine and further comprises quercetin, and a pharmaceutically acceptable carrier. In one aspect, the combination comprises luteolin and caffeine and further comprises apigenin, and a pharmaceutically acceptable carrier.

### EXAMPLES

The following examples are to be considered as non-limiting examples. In these examples, we demonstrate the effect of several orally available small molecule compounds on splicing of pre-mRNAs that carry splice-acceptor site mutations. The tested compounds have been shown to have a beneficial effect on either mRNA stability and/or pre-mRNA splicing but none have been shown to result in effect on splice-acceptor site mutations. We here showed that some derivatives of xanthine, for example, caffeine, and the plant-derived flavonoid luteolin are able to significantly correct the splicing of *AGA* pre-mRNA mutated at position -2 preceding exon 2 in a minigene splicing assay. In addition, caffeine and luteolin significantly increased the amount of correctly spliced AGA mRNA and AGA enzyme activity in patient fibroblasts homozygous for the said gene defect. Importantly, our data also show that the splicing of the *TPP1* gene deficient in another lysosomal storage disorder, cLINCL, can be improved using the same substances. Our findings demonstrate that these substances can provide a relevant therapeutic intervention for diseases that result from splice-acceptor site mutations. Small orally available substances can therefore provide either a standalone systemic therapy or a therapy in combination with gene therapy, enzyme replacement therapy or therapy with oligonucleotides targeting the splice defects.

### Materials and Methods

### Cell culture

HEK293T (human embryonic kidney cells) cells were cultured in Dulbecco's modified Eagle's medium (DMEM) with high glucose, 10% fetal calf serum (FCS), 1 % penicillin/streptomycin (all from Gibco, Thermo Fisher Scientific, Germany). AGA knockout HEK293T cells without endogenous AGA expression were created by Crispr/Cas9 and have been described in Kapahnke, M.; Banning, A.; Tikkanen, R. Random Splicing of Several Exons Caused by a Single Base Change in the Target Exon of CRISPR/Cas9 Mediated Gene Knockout. Cells 2016, 5. Primary skin fibroblasts carrying the homozygous intronic mutation c.128-2A>G in the *AGA* gene were obtained from a 7-year-old female Australian patient of Indian heritage at Murdoch Children's Research Institute (Victoria, Australia) and have been described in Banning, A.; et al. Biochim Biophys Acta Mol Basis Dis 2018, 1864, 668-675. The parents provided a signed informed consent for the biopsy, fibroblasts culture, storage, enzymatic and molecular analyses, and the procedures were approved by the Murdoch Children's Research Institute (Victoria, Australia). Immortalized, hTERT-transformed normal human skin fibroblasts were used as control [(Banning, A.; et al. Identification of Small Molecule Compounds for Pharmacological Chaperone Therapy of Aspartylglucosaminuria. Sci Rep 2016, 6, 37583]. All fibroblasts were cultured in DMEM (high glucose), 10% FCS, 1 % penicillin/streptomycin, 1 % non-essential amino acids and 1 % sodium pyruvate. All cells were grown at 8% CO₂ and 37°C.

### Minigene constructs and transfection

The AGA-minigene-pcDNA3 and AGA-minigene-Fluc-pcDNA3 constructs contain approximately 2900 bp of PCR-amplified AGA genomic sequence spanning exons 1-3 with the mutation c.128-2A>G, either containing the firefly luciferase coding sequence at the 3' end or without luciferase. The TPP1-minigene-pcDNA3 constructs with or without luciferase contain approximately 800 bp of *TPP1* genomic sequence spanning exons 5-7 with the mutation c.509-1G>C. The firefly luciferase coding sequence (Fluc) was PCR amplified from pGL3-basic (Promega, Walldorf, Germany, GenBank number U47295) without the ATG start codon and was cloned in-frame 3' of the *AGA* exon 3 or *TPP1* exon 7, respectively. All constructs were verified by DNA sequencing (Seqlab, Göttingen, Germany). Primer sequences for cloning are shown in Table 1.

**Table 1. Primer sequences for cloning of the AGA and TPP1 minigene constructs. All sequences are shown in 5' to 3' direction.**

| **Primer name** | **Primer sequence** | **SEQ ID No** |
|---|---|---|
| AGA-Minigene fwd HindIII | CTATAAAGCTTAGGGACGCCTGAGCGAACCC | 1 |
| AGA-Minigene rev XhoI | CTATACTCGAGCT- GACTCTCCTACTAAAAGTGTGT | 2 |
| TPP-IVS-Minigene fwd BamHI | CTAGGATCCAT- GCAAGCAGAGCTGCTGCTCCCTG | 3 |
| TPP-IVS-Minigene rev XhoI | CTATACTCGAGCAGGGCTACTGTAGAC- CCAGGTG | 4 |
| Luciferase fwd XhoI | CTATACTCGAGAAGACGCCAAAAACATAAA- GAAAGG | 5 |
| Luciferase rev XbaI | CTATATCTAGATTACACGGCGATCTTTCCGCC | 6 |

For transient transfection, cells were seeded onto 12-well plates on the day before transfection. For transfections, 400 ng of expression plasmid DNA and 50 ng of pRL-TK Renilla luciferase control reporter vector (Promega) were transfected using MACSfectin^{™} (Miltenyi Biotec, Bergisch Gladbach, Germany) according to the manufacturer's protocol. Fibroblasts were transfected with Neon Transfection System (Invitrogen). The following day, the cells were transferred onto 24-well plates, treated with specific substances and harvested after 24 h of treatment in lysis juice for firefly and renilla luciferase assay (PJK GmbH, Kleinblittersdorf, Germany). Determination of firefly and renilla luciferase activity was performed with a Tecan infinite M200 plate reader using 20 µl of lysate and 85 µl of beetle or renilla juice (PJK) as reagents. Relative luciferase activity was calculated by dividing the firefly luciferase activity by the renilla luciferase activity.

### 2.3. Treatment of patient fibroblasts with recombinant AGA or splicing modulating compounds

Fibroblasts were seeded into 6-well plates and treated with the following compounds: caffeine, theophylline, theobromine, pentoxifylline, doxofylline(1-7.5 mM, solvent H₂O; TCI, Eschborn, Germany), luteolin (10-50 µM, solvent DMSO; Biozol, Eching, Germany) or kinetin (10-100 µM, solvent DMSO; TCI, Eschborn,Germany) for 24 h (RNA) or 48 h (protein lysate). Cells were harvested in lysis buffer (50 mM Tris pH 7.4, 150 mM NaCl, 2 mM EDTA, 1% NP-40), supplemented with protease inhibitor cocktail (Sigma-Aldrich). Protein concentrations in the lysates were determined according to the Bradford method.

### 2.4. Antibodies

A mouse monoclonal antibody against luciferase (sc-74548, Santa Cruz Biotechnology, Heidelberg, Germany) was used to detect firefly luciferase in Western blots. A mouse monoclonal antibody against GAPDH was purchased from Abcam (Cambridge, UK).

### Enzyme activity measurements

In most experiments, AGA activity was measured fluorimetrically as described in Banning, A.; et al. Sci Rep 2016, 6, 37583. Reaction mixtures contained 10-20 µl cell lysate or lysis buffer only for a blank and 20 µl of Asp-AMC (L-Aspartic acid β-(7-amido-4-methylcoumarin), 50 µM in Mcllvain's phosphate-citrate buffer pH 6.5. The samples were incubated for 24 h at 37°C, after which the reaction was stopped by addition of 200 µl Mcllvain's buffer pH 4.5. Triplicate samples were measured with a Tecan infinite M200 plate reader (355 nm excitation and 450 nm emission). AGA activity in samples that had been treated with luteolin were measured photometrically with the Morgan-Elson-assay using 2-acetamido-1-ß-(L-as-partamido)-1,2- dideoxy-ß-D-glucose (AADG, Santa Cruz) as substrate, because of the fluorescence quenching properties of luteolin. In short, 12.5 µl of cell lysate were mixed with 100 nmol AADG, 3.35 µl 0,5 M KH₂PO₄, pH8, and 0.9% NaCl to reach a total volume of 25 µl. Samples were incubated at 37°C for 24 h. Reactions were stopped by adding 53.5 µl of 0,8 M borate buffer pH9.1 and boiling for 5 minutes at 100°C. Five volumes of DMAB reagent (10 mg DMAB/ml glacial acetic acid) were added and samples incubated for 20 minutes at 37°C until a purple color was visible. The liberated N-acetylglucosamine was measured at 585 nm. N-acetylglucosamine (25-1000 µM) was used as standard.

### Quantitative Real-Time PCR

For qPCRs, total RNA was isolated with peqGOLD TriFast^{™} (VWR, Darmstadt, Germany) and included a DNAse step. Total RNA (1-3 µg) were reverse transcribed with 150 fmol oligo(dT) primers and M-MuLV reverse transcriptase (NEB, Frankfurt, Germany) in a total volume of 45 µL. Real-time quantitative PCRs were performed using the CFX Connect Real-Time PCR Detection System (Bio-Rad, Munich, Germany). Annealing temperature was 60°C for all primers. The reactions were done as duplicates with 0.8 µL cDNA in a total volume of 10 µL using iTaq-TMUniversal SYBR Green Supermix (Bio-Rad). PCR products were quantified with the ACt-method. For normalization, the mean of the reference genes B2M, Rpl13a, Ywhaz and TBP was used. The primer sequences are shown in Table 2.

**Table 2. Sequences of primers used for the quantitative real-time PCR. All sequences are shown in 5' to 3' direction.**

| **Primer name** | **Primer sequence 5' to 3'** | **SEQ ID No** |
|---|---|---|
| B2M-fwd | AGATGAGTATGCCTGCCGTGTG | 7 |
| B2M-rev | TGCGGCATCTTCAAACCTCCA | 8 |
| Rpl13a-fwd | CCTGGAGGAGAAGAGGAAAGAGA | 9 |
| Rpl13a-rev | TTGAGGACCTCTGTGT A TTTGTCAA | 10 |
| Ywhaz-fwd | AGGTTGCCGCTGGTGATGAC | 11 |
| Ywhaz-rev | GGCCAGACCCAGTCTGATAGGA | 12 |
| TBP-fwd | GACTATTGGTGTTCTGAATAGGC | 13 |
| TBP-rev | GGAATCCCTATCTTTAGTCCAAT | 14 |
| AGA exon2 fwd | CGGCTCTGTAGGCTTTGGAGGA | 15 |
| AGA exon3 rev | CCAGTACTTTCCGTGCCACACC | 16 |
| AGA exon 7/8 fwd | ATGGCCGTGTAGGAGACTCACC | 17 |
| AGA exon 8/9 rev | ACAGCTTGGTAGCTTGGCAGGA | 18 |
| TPP1 exon 6/7 fwd | CTGTGCCCAGTTCCTGGAGC | 19 |
| pCDNA3-rev | GGCAACTAGAAGGCACAGTC | 20 |

### Immunofluorescence

Cells were seeded on glass coverslips and cultured for at least 2 days. For staining of acidic cell compartments, the cells were incubated with 5 µM Lysotracker-Red (Invitrogen) in cell culture medium for 30 min. All cells were fixed in 4% paraformaldehyde for 10 min at room temperature. The samples were analyzed with a Zeiss LSM710 Confocal Laser Scanning Microscope (Carl Zeiss, Oberkochen, Germany).

### Statistical analysis

All experiments were performed at least three times. Data are expressed as mean ± SD. Statistical comparisons between groups were made using Student's t-tests, 1way or 2way ANOVA (Analysis of Variance), as appropriate, using the GraphPad Prism 5 software (San Diego, CA, USA). Values of *p* < 0.05 were considered significant (*) while values of *p* < 0.01 were considered very significant (**) and *p* < 0.001 extremely significant (***).

### Results

### An AGU patient with a homozygous splice acceptor site mutation

A seven-year-old female of Indian origin with a developmental delay was presented at Murdoch Children's Research Institute (Victoria, Australia)[Banning, A.; et al. Biochim Biophys Acta Mol Basis Dis 2018, 1864, 668-675]. Metabolic analysis of the urine showed a substantial increase of glycoasparagines (mainly aspartylglucosamine), suggesting AGU as the underlying disease. Sequence analysis of the *AGA* gene revealed a homozygous point mutation at the 3' splice-acceptor site of the intron 1 (c.128-2A>G). The consensus splice motif AG at the end of the intron 1 is exchanged into GG. Therefore, it can be predicted that exon 2 is spliced out together with introns 1 and 2, resulting in a frame-shift and an early translation termination (Figure 1). This variant can be classified as a potentially harmful one, but it has not been reported in an AGU patient before. The parents of the patient are not aware of being related with each other, suggesting a non-consanguineous origin of the mutated alleles.

A skin biopsy of the patient was performed, and fibroblast cultures were established for further analysis. The fibroblasts were used and have been described in a previous study of ours [Id.] The AGA enzyme activity in the fibroblasts cultures was 8.3% (SD 6.3%) of control fibroblasts (Figure 2A), consistent with AGU. Lysotracker staining of the fibroblasts revealed an enlarged lysosomal compartment, typical of lysosomal storage disorders (Figure 2B). Treatment of the patient fibroblasts with recombinant, purified AGA [Banning, A.; et al., Sci Rep 2016, 6, 37583] resulted in normalization of the AGA enzyme activity (Figure 2C). A qRT-PCR with primer pairs for *AGA* exons 2 and 3 or 7 and 8 showed a reduction of the *AGA* mRNA in the patient cells (Fig. 2D). However, while the primer pair for exons 7 and 8 suggested a reduction to 39.0% of the mRNA level in control fibroblasts, the reduction observed with the primer pair for exons 2 and 3 was more pronounced (down to 9.4% of the control) suggesting that a splicing defect of exons 2 and/or 3 might be present.

Since the above data are suggestive of a splicing defect, we characterized the *AGA* splicing variants in the patient cells. The *AGA* open reading frame was PCR-amplified from cDNA, cloned into a plasmid vector, and six cDNA clones were sequenced (Figure 3). The WT sequence and the resulting protein translation are shown in Figure 3a. The most abundant (four out of six clones analyzed) *AGA* splicing isoform in the patient cells exhibits an exclusion of the exon 2 (Figure 3b), as expected due to the mutation in the acceptor splice-site preceding exon 2. In addition, single clones that contained either a deletion of exons 2 and 3 (Figure 3c) or exons 2 and 6 (Figure 3d) were also detected. Clones with out-of-frame deletions of exon 2 or exons 2+6 result in an early STOP codon and a predicted highly truncated protein. However, a deletion of exons 2 and 3 results in normalization of the reading frame from exon 4 onwards, and the predicted protein would exhibit a deletion of 89 amino acids in the N-terminal half of the α subunit, but otherwise a normal amino acid sequence.

### A luciferase minigene assay for testing substances that affect AGA splicing

Numerous natural substances exhibit potential as splicing enhancers due to their capability to enhance mRNA stability or to affect splicing directly [Fujita, K.I.; et al. Int J Mol Sci 2020, 21]. For testing such substances, we developed a minigene assay that is based on a DNA construct containing AGA exons 1, 2 and 3, together with the introns 1 and 2, followed by the firefly luciferase coding sequence (Figure 4). This construct contains the splice acceptor site mutation -2A>G in intron 1. If exon 2 is spliced out, altered reading frame in exon 3 produces an early STOP codon and prevents the expression of luciferase. However, if all three exons are correctly spliced, the luciferase ORF is translated in-frame as a fusion protein with the AGA protein sequence coded by exons 1-3. Thus, the effect of a treatment of cells with substances that enhance splicing can be measured using luciferase activity, measured as emission of visible light.

### Xanthine derivatives enhance the correct splicing of the mutated AGA mRNA

Natural substances that are derived from xanthine, such as caffeine and theophylline, have been shown to stabilize mRNAs and to exhibit splicing enhancer activity [Keeling, K.M.; et al. PLoS One 2013, 8, e60478]. Using the *AGA* minigene assay, we tested the capability of five xanthine derivatives to dose dependently enhance the splicing of the *AGA* minigene containing the splice variant (Figure 5). Four different concentrations of the substances (1 mM - 7.5 mM) were tested in our AGA knockout HEK293T cell line [Kapahnke, M., et al., Cells 2016, 5] transfected with the *AGA* minigene construct. The cells were treated with the substances for 24 h, lysed, and the firefly and renilla luciferase activities were measured. Caffeine (1,3,7-trimethylxanthine) treatment resulted in a dose dependent, significant (at 5 mM and 7.5 mM) increase in the luciferase activity as compared to untreated cells (Figure 5a). Similarly, 7.5 mM theophylline (1,3-dimethylxanthine), doxofylline (7-(1,3-dioxolan-2-ylmethyl)-1,3-dimethylpurine-2,6-dione) and pentoxifylline (3,7-dimethyl-1-(5-oxohexyl) xanthine) also significantly increased luciferase activity (Figure 5b -d), whereas theobromine (3,7-dimethylxanthine) resulted in non-significant changes (Figure 5e). Figure 5f shows a direct comparison of the substances at 7.5 mM, with caffeine showing the highest increase but also the largest variation. Figure 5g shows a Western Blot for the mini-AGA-luciferase fusion protein expression in cells treated with the 7.5 mM compounds.

To verify that the treatment of the cells resulted in inclusion of exons 2 and 3 at the mRNA level, a second minigene construct without the luciferase coding sequence was cloned in the pCDNA3 vector (Figure 6a). AGA knockout HEK293T cells were transfected with this construct and treated with the above substances. RNA was isolated from the cells, reverse transcribed to cDNA, and a qRT-PCR with a fwd primer residing in exon 2 and a reverse primer in the plasmid vector (3'-untranslated region of the resulting RNA) was performed. Thus, amplification of a PCR product of 271 bp is only observed when both exons 2 and 3 are present in the mRNA. Figure 6b shows that 7.5 mM caffeine resulted in a significant, almost 20-fold increase in the correctly spliced product. Pentoxifylline and theophylline also significantly increased the correct splicing up to about 5 to 10-fold, whereas doxofylline and theobromine did not produce significant changes in the splicing. This assay also showed a good dose dependency (Figure 6c), and there was a good correlation (R²= 0.95) between the mRNA and luciferase-based minigene assays (Figure 6d).

Since the genetic background can have a substantial effect on splicing, we also tested the minigene splicing assays in the AGU patient fibroblasts that were transfected with the AGA-luciferase minigene constructs and then treated with 7.5 mM xanthine derivatives as above (Figure 7). As with the HEK293T cells, caffeine and theophylline showed a significant, dose dependent increase in the luciferase readout (Figure 7a-c), and pentoxifylline also significantly increased the luciferase activity at 7.5 mM, whereas theobromine and doxofylline failed to do so (Figure 7a).

The above data pointed to caffeine as the most efficient splicing enhancer for the patient variant studied here. We thus tested the effect of the above substances on splicing and AGA enzyme activity in patient fibroblasts (Figure 8). Caffeine treatment resulted in significant increase of the AGA enzyme activity up to 23.4% of the control fibroblast activity (Figure 8a), whereas all other substances failed to increase the AGA activity to a significant degree (Figure 8b). Consistent with the enzyme activity data, the endogenous AGA mRNA splicing products that contain exons 2 and 3 were significantly increased to a level that corresponds to the untreated control fibroblasts, albeit with a high SD (Figure 8c). Interestingly, caffeine treatment also resulted in an increase in the AGA mRNA in control fibroblasts, which is consistent with the general mRNA stabilizing effect of caffeine.

### Plant-derived bioactive compounds enhance the splicing of the AGA mRNA

Although caffeine treatment looked quite promising according to our data above, we tested further substances that may enhance splicing, since caffeine, at least in large amounts, may be a suboptimal substance, specifically, for the treatment of pediatric patients. Luteolin (3',4',5,7-tetrahydroxyflavone) is a plant-derived flavonoid that has been shown to enhance splicing at non-canonical, mutated splice-acceptor sites [Chiba, M., et al., Chem Biol Drug Des 2016, 87, 275-282]. Kinetin (6-furfuryl-adenine) is a member of the cytokinin plant hormone family, and it has been shown to enhance the splicing in the *IKBKAP* gene carrying a splice-donor site mutation [Hims, M.M.; et al. J Mol Med (Berl) 2007, 85, 149-161; Slaugenhaupt, S.A.; et al. Hum Mol Genet 2004, 13, 429-436].

We tested the effect of luteolin and kinetin on the splicing of the AGA mRNA with the c.128-2A>G variant. AGA knockout HEK 293T cells were transfected with the AGA-luciferase minigene, treated with 10 µM - 100 µM kinetin or 10 µM - 50 µM luteolin, and the luciferase activity was measured in cell lysates (Figure 9a). Treatment with 100 µM kinetin resulted in a significant (about two-fold) increase in luciferase activity, whereas the lower concentrations showed only minor effects. In contrast, all luteolin concentrations resulted in a significant increase in luciferase activity, with 25 µM luteolin being the most efficient concentration (about 5-6-fold increase). Consistently, a strong luciferase expression in Western blot was only observed in luteolin treated lysates (Figure 9b). In contrast, the qPCR-based minigene assay showed that both luteolin and kinetin increased the amount of AGA mRNA products containing exon 2 (Figure 9c).

When the patient fibroblasts were treated with luteolin or kinetin, the endogenous AGA mRNA with exon 2 was significantly increased by luteolin (about 2.5-fold), whereas kinetin treatment showed no significant increase (Figure 10a). Due to these encouraging data, the AGA activity was measured in patient fibroblasts treated with 25 µM luteolin (Figure 10b). Since luteolin acts as a quencher in our standard fluorescence-based AGA activity measurement [Schoefer, L.; Braune, A., FEMS Microbiol Lett 2001, 204, 277-280, and our own observations], we used a colorimetric activity assay [Tikkanen, R.; et al., DNA Cell Biol 1995, 14, 305-312] that is less sensitive but not subject to quenching by luteolin. Indeed, luteolin treatment resulted in a significant increase in the AGA activity in the patient fibroblasts (Figure 10b).

The mechanisms of action of caffeine and luteolin on splicing are not identical, and a combination therapy with both substances could facilitate the use of lower concentrations of especially caffeine in the patient. Thus, patient fibroblasts were treated with 25 µM luteolin, 2.5 mM caffeine, or a combination thereof. Indeed, combination treatment with both substances resulted in a substantially higher AGA activity than either luteolin or caffeine alone (Figure 10c).

### Xanthine derivatives and luteolin correct the splicing of a common cLINCL/TPP1 variant

Splice-site mutations are relatively rare in AGU, whereas in some other lysosomal disorders, they represent the most frequent disease-causing variants found in patients. Classic late neuronal ceroid lipofuscinosis (cLINCL), also known as CLN2 disease, is caused by mutations in the *TPP1* gene that encodes for the soluble lysosomal enzyme tripeptidyl peptidase 1 (TPP1). One of the most common disease-causing variants in cLINCL is a splice-acceptor site mutation c.509 -1G>C that is present in 27% of the reported patient alleles [Gardner, E.; et al., Hum Mutat 2019, 40, 1924-1938]. In this variant, the splice-acceptor consensus sequence at the end of intron 5 has been abolished, thus potentially resulting in the exclusion of exon 6 upon splicing of the *TPP1* pre-RNA (Figure 11a). Loss of exon 6 produces a frameshift and early termination of translation after 99 amino acids encoded by exon 7.

Due to the encouraging data obtained with our AGU splice-site variant, we constructed a *TPP1* luciferase minigene that contains exons 5, 6 and 7 together with the introns 5 and 6 (Figure 11a) and the firefly luciferase coding region. The splice-site variant -1G>C was introduced at the end of intron 5. Upon exclusion of exon 6, which is the most likely alteration, no luciferase activity is observed due to the frameshift and premature stop codon. Other altered splicing products are possible, but they would not result in a luciferase signal. If the splicing can be corrected, and exon 6 is included, luciferase activity can be measured (Figure 11a).

Significant increase of luciferase signal was detected with 7.5 mM caffeine, theophylline, pentoxifylline, and with 25 µM luteolin (Figure 11b). Luciferase expression was verified by western blot (Figure 11c) and was well in accordance with the activity readouts from Figure 11b.

Analogously to our approach with the AGA splicing assay, we also constructed a *TPP1*

## Claims

1. A pharmaceutical composition comprising a methylxanthine derivative, such as caffeine, pentoxifylline or theophylline, and/or a plant-derived flavonoid or a combination thereof for use in the treatment of a genetic disease determined to be caused by a splice-acceptor site mutation in an intron.

2. The pharmaceutical composition according to claim 1, wherein the splice-acceptor site mutation is in the position -1 or -2 of the acceptor site of the intron.

3. The pharmaceutical composition according to claim 1 or 2, wherein the splice-acceptor site mutation is in the position -1 of the acceptor site of the intron and has altered the -1 position from nucleotide G into C, A or T.

4. The pharmaceutical composition according to claim 1 or 2, wherein the splice-acceptor site mutation is in the position -1 of the acceptor site of the intron and has altered the -1 position from nucleotide G into C or A.

5. The pharmaceutical composition according to claim 1 or 2, wherein the splice-acceptor site mutation is in the position -2 of the acceptor site of the intron and has altered the -2 position from nucleotide A into G, C or T.

6. The pharmaceutical composition according to claim 1 or 2, wherein the splice-acceptor site mutation is in the position -2 of the acceptor site of the intron and has altered the -2 position from nucleotide A into G or C.

7. The pharmaceutical composition according to any of the preceding claims, wherein the encoded protein is an enzyme, such as a lysosomal enzyme, such as aspartylglucosaminidase or tripeptidyl peptidase 1.

8. The pharmaceutical composition according to any one of claims 1-6, wherein the encoded protein is a structural protein.

9. The pharmaceutical composition according to any one of claims 1-6, wherein the encoded protein is a membrane protein.

10. The pharmaceutical composition according to any one of the preceding claims, wherein the methylxanthine derivative is caffeine.

11. The pharmaceutical composition according to any one of claims 1-4, wherein the plant-derived flavonoid is luteolin.

12. The pharmaceutical composition according to any one of the preceding claims, comprising a combination of caffeine and luteolin.
